# EUROPEAN PATENT SPECIFICATION

(11) **EP 0 662 150 B1**
(45) Date of publication and mention of the grant of the patent: **18.12.2002**
(21) Application number: 93921337.7
(22) Date of filing: 01.09.1993
(51) Int. Cl.: C07K 14/81, C12Q 1/68, G01N 33/48, G01N 33/50, C12N 5/00, C12N 15/00, A61K 39/00, C07H 21/04

(54) **MASPIN, A NOVEL SERPIN WITH TUMOR SUPPRESSING ACTIVITY**
MASPIN- EIN NEUES SERPIN MIT TUMOR UNTERDRÜCKENDER AKTIVITÄT
MASPIN, NOUVEAU SERPIN A ACTIVITE ANTITUMORALE

(30) Priority: 01.09.1992 US 938823
(43) Date of publication of application: 12.07.1995
(73) Proprietor: DANA-FARBER CANCER INSTITUTE, INC., Boston, MA 02115 (US)
(72) Inventor: SAGER, Ruth, Brookline, MA 02146 (US); ZOU, Zhiqiang, Gaithersburg Maryland 20878 (US); ANISOWICZ, Anthony, Massachusetts 02165 (US)
(74) Representative: Baldock, Sharon Claire
(86) International application number: US9308322
(87) International publication number: WO94005804

(56) References cited:
- WO-A-92/15602
- US-A- 4 888 278
- Science, Volume 246, issued 15 December 1989, RUTH SAGER, "Tumor Suppressor Genes: the Puzzle and the Promise", pages 1406-1412, entire document.
- Proc. Nat. Acad. Sci., Volume 88, Number 7, issued April 1991, S.W. LEE et al., "Positive Selection of Candidate Tumor-Suppressor Genes by Subtractive Hybridization", pages 2825-2829, entire document.
- Virology, Volume 160, issued 1987, R. CATTANEO et al., "Altered Ratios of Measles Virus Transcripts in Diseased Human Brains", pages 523-526, entire document.

## Description

This application is a continuation-in-part of currently pending USSN 07/938,823 (herein incorporated by reference), which was filed September 1, 1992, and is commonly owned with the present application, and which in turn is a continuation-in-part of USSN 07/844,296, filed February 28, 1992, now abandoned, which in turn is a continuation-in-part of USSN 07/662,216, filed February 28, 1991, now abandoned.

### Statement as to Federally Sponsored Research

The invention described herein was made in part with the support of the U.S. Government (NIH grant nos. PO1 CA22427 and OIG CA39814 to Dr. Ruth Sager). The U.S. Government therefore has certain rights in the invention.

### Background of the Invention

The field of the invention is tumor suppressor genes.

Cancer at the cellular level is characterized by the disruption of multiple regulatory mechanisms resulting from multiple genetic changes. The search for specific genes with major cancer-related effects has focussed on two fundamental processes: control of proliferation, and control of invasion and metastatic spread. Both processes are complex, and the relevant cancer-related changes in gene expression involve both increases and decreases in the activity of particular proteins.

Metastatic spread occurs when primary tumor cells invade into lymphatics and blood vessels, and disseminate to distant organs (Fidler et al., J. Natl. Cancer Inst. 82:166, 1990; Liotta et al., Cancer Res. 51:5054, 1991; Nicolson, Semin. Cancer Biol. 2:143, 1991; and Chen, Current Opin. Cell Biol. 4:802, 1992). The multiple steps involved in metastasis include proteolytic attack on the basement membrane and extracellular matrix (ECM), adhesion to endothelial cells leading to intravasation, and later extravasation from the circulatory system into tissues such as lung and bone in which the tumor cells are able to proliferate. In normal cells, these processes of invasion and metastasis are blocked by an intricate array of genetically programmed regulatory mechanisms. Overcoming these protective barriers to invasion and metastasis requires multiple changes in gene expression, resulting in gain or loss of gene functions that contribute to tumor progression.

Increased proteolytic activity augments invasion, as evidenced by the increased activity of serine proteases (Testa et al., Cancer Metastasis Rev. 9:353, 1990; Dano et al., Adv. Cancer Res. 44:139, 1985; Foekens et al., Cancer Res. 52:6101, 1992; Ossowsky, Cancer Res. 52:6754, 1992; Sumiyoshi, Int. J. Cancer 50:345, 1992; Duffy et al., Cancer Res. 50:6827, 1992; and Meissauer et al., Exp. Cell Res. 192:453, 1991, metalloproteases (Birkedal-Hansen (ed) Proceedings of the Matrix Metalloporteinase Conference, Destin, Florida, Gustav Fischer Verlag, 1990; Matrisian et al., Am. J. Med. Sci. 302:157, 1991; Stetler-Stevenson, Cancer Metastasis Rev. 9:289, 1990; Declerck et al., Cancer Res. 52:701, 1992; Bassett, Nature 348:699, 1990; Wolf et al., Proc. Natl. Acad. Sci. USA 90:1843, 1993; and Sato et al., Oncogene 7:77, 1992), and cathepsins (Rochefort et al., Cancer Metast. Rev. 9:321, 1990; and Kobayashi et al., Cancer Res. 52:3610, 1992) in invasive tumor cells. The principal serine proteases known to be associated with tumor invasion mediate the plasminogen activation cascade. In this pathway, plasminogen is converted by plasminogen activators to plasmin, which is a wide-spectrum serine protease that degrades many components of the ECM directly, or indirectly via the activation of metalloproteases. The activity of the plasminogen activators is negatively regulated by plasminogen activator inhibitory proteins: PAI-1, PAI-2, and protease nexins (Chen, Current Opin. Cell Biol. 4:802, 1992).

The elevated expression of uPA (urokinase plasminogen activator) in breast carcinomas and other cancers has been reported by numerous investigators since the 1970's (Testa et al., Cancer Metastasis Rev. 9:353, 1990; Dano et al., Adv. Cancer Res. 44:139, 1985; Foekens et al., Cancer Res. 52:6101, 1992; Ossowsky, Cancer Res. 52:6754, 1992; Sumiyoshi, Int. J. Cancer 50:345, 1992; Duffy et al., cancer Res. 50:6827, 1992; and Meissauer et al., Exp. Cell Res. 192:453, 1991; Heidtmann et al., Cancer Res. 49:6960, 1989; Sumiyoshi et al., Thromb Res. 63:59, 1991; Reilly et al., Int. J. Cancer 50:208, 1992, Cajot et al., Proc. Natl. Acad. Sci. USA 87:6939, 1990; Foucre et al., Br. J. Cancer 64:926, 1991; Shirasuna et al., Cancer Res. 53:147, 1993; and Janicke et al., Br. Can. Res. & Treat. 24:195, 1993). More recently, it has been shown that PAI-1 and PAI-2 are also elevated in malignancy (Sumiyoshi, Int. J. Cancer 50:345, 1992; Heidtmann et al., Cancer Res. 49:6960, 1989; Sumiyoshi et al., Thromb Res. 63:59, 1991; Reilly et al., Int. J. Cancer 50:208, 1992, Cajot et al., Proc. Natl. Acad. Sci. USA 87:6939, 1990; Foucre et al., Br. J. Cancer 64:926, 1991; Shirasuna et al., Cancer Res. 53:147, 1993; and Janicke et al., Br. Can. Res. & Treat. 24:195, 1993). These findings are inconsistent with the simple paradigm of protease/antiprotease balance in normal cells and its imbalance in tumor cells, thus confusing the issue of how effective uPA may be in metastatic dissemination. Recent studies of the uPA receptor and its importance in modulating uPA activity (Testa et al., Cancer Metastasis Rev. 9:353, 1990; Vassalli et al., J. Cell Biol. 100:86, 1985; and Lund et al., EMBO J. 10:3399, 1991) have indicated further levels of regulation. Thus, although it has been clearly established that uPA is capable of degrading components of the basement membrane and ECM, and that it is often elevated in advanced breast cancer, its precise role in breast cancer invasion remains to be established. Similarly, the importance of PAI-1 and PAI-2 in inhibiting breast cancer invasion is not clearly established (Testa et al., Cancer Metastasis Rev. 9:353, 1990).

The matrix metalloproteases (MMPs) include collagenases and stromelysins. The type IV collagenases (gelatinases), in particular the 72 kDa form, are active in tumor invasion, as indicated by elevated levels in aggressive human tumors (Stetler-Stevenson, Cancer Metastasis Rev. 9:289, 1990). The tissue inhibitors of metalloproteinase activity, TIMP-1 and TIMP-2, target the type IV collagenases, with TIMP-2 interacting exclusively with the 72 kDa form (Stetler-Stevenson et al., Annu. Rev. Cell Biol. 9:541, 1993). Stromelysins-1 (transin) and -2 have been associated with tumor progression in rodent systems, whereas a smaller molecule called PUMP has been identified in human tumor cells (Matrisian et al., Am. J. Med. Sci. 302:157, 1991). Extensive studies of stromelysin-3 have shown a strong correlation with advanced breast cancer (Bassett, Nature 348:699, 1990; Wolf et al., Proc. Natl. Acad. Sci. USA 90:1843, 1993). This protease is secreted by stromal fibroblasts that are proximal to invasive primary breast carcinomas, and not by the epithelial tumor cells, showing the importance of cell-cell interactions in tumorigenic mechanisms.

### Summary of the Invention

Disclosed herein is a new gene, originally isolated by subtractive hybridization, that is involved in protection against a primary step in the metastatic cascade. The gene, called maspin, encodes a novel serine protease inhibitor expressed in normal mammary epithelial cells in culture and in the normal breast. Its expression decreases during progression from well-differentiated to poorly differentiated primary carcinomas, and is absent in most lymph node and distant metastatic lesions. The inferred structure of the protein is consistent with serine protease inhibitor activity. Functional studies indicate that the protein has tumor suppressing and invasion suppressing activity.

The invention thus includes an isolated DNA encoding a polypeptide substantially identical to maspin (i.e., having at least 90% sequence identity to SEQ ID NO:2, with any amino acid substitutions preferably being conservative). The isolated DNA preferably contains a DNA sequence which hybridizes under stringent conditions (as defined below) with the DNA sequence of SEQ ID NO:1, or the complement thereof, and may contain the sequence of SEQ ID NO: 1. It is preferably incorporated into a vector (a virus, phage, or-plasmid) which can be introduced by transfection or infection into a cell. The vector preferably includes one or more expression control sequences, in which case the cell transfected by the vector is capable of expressing the polypeptide. By "isolated DNA" is meant a single- or double-stranded DNA that is free of the genes which, in the naturally-occurring genome of the animal from which the isolated DNA is derived, flank the maspin gene. The term therefore includes, for example, either or both strands of a cDNA encoding maspin or an allelic variant thereof; a recombinant DNA which is incorporated into a vector, into an autonomously replicating plasmid or virus, or into the genomic DNA of a prokaryotic or eukaryotic cell; or a genomic DNA fragment (e.g., produced by PCR (polymerase chain reaction] or restriction endonuclease treatment of human or other genomic DNA). It also includes a recombinant DNA which is part of a hybrid gene encoding additional polypeptide sequence.

Stringent conditions for both DNA/DNA and DNA/RNA hybridization assays are as described by Sambrook et al., Molecular *Cloning, A Laboratory Manual*, 2nd Ed., Cold Spring Harbor Laboratory Press, Cold Spring Harbor, KY, 1989. For example, see page 7.52 of Sambrook et al.

Also within the invention is an isolated DNA at least 30 nucleotides in length (preferably at least 100, more preferably at least 500), including (a) a strand which hybridizes under stringent conditions to a DNA having the sequence of SEQ ID NO: 1, (b) the complement thereof, or (c) a double stranded DNA including both (a) and (b). Multiple copies of this isolated DNA (useful, for example, as a hybridization probe or PCR primer) can be produced by recombinant means, by transfecting a cell with a vector containing this DNA.

The invention also includes a purified preparation of maspin protein (SEQ ID NO:2), or a fragment of maspin that is an antigenic polypeptide containing from 10 to 374 amino acid residues of maspin (preferably at least 12, more preferably at least 14, and most preferably at least 18 (e.q., 20 or more), which polypeptide fragment contains an epitope of maspin such that an antibody raised against the fragment (or against a conjugate of the polypeptide and keyhole limpet hemocyanin) forms an immune complex with maspin itself. Such an antibody may be either polyclonal or monoclonal, and is generated by standard methods including the step of immunizing an animal with an antigen containing an antigenic portion of maspin. Also within the invention are hybrid polypeptides containing (1) maspin or an antigenic fragment thereof, covalently attached to (2) a second polypeptide. Such hybrid polypeptides can be made by any of a number of standard techniques well known to those of ordinary skill, including recombinant methods, in which case the covalent attachment is a peptide bond, or chemical conjugation, in which case the covalent attachment is another type of bond, such as a disulfide bond. Linking maspin or an antigenic fragment thereof to a second polypeptide provides a means for readily isolating the hybrid from a mixture of proteins, by the use of an affinity column to which the second polypeptide (e.g., glutathione transferase) binds directly. Such hybrid polypeptides may also have the advantage of increased immunogenicity relative to maspin or the maspin fragment, so that antibodies are more readily obtained.

Both the isolated DNAs of the invention and the antibodies of the invention are useful in diagnostic methods for detecting carcinomas, or for staging a carcinoma, where the suspected carcinoma is derived from a type of cell which normally expresses the maspin gene to a significant and easily detectable degree (e.g., mammary epithelial cells). One such diagnostic method includes the steps of providing a test cell (e.g., in the form of a tissue section or a cell preparation) from a given type of epithelial tissue; contacting the mRNA of the test cell with a nucleic acid probe containing a sequence antisense to (i.e., complementary to the sense strand of) a segment of SEQ ID NO: 1, which segment is at least 30 (preferably at least 40, more preferably at least 100), nucleotides in length; and comparing (1) the amount of hybridization of the probe to the mRNA of the test cell, with (2) the amount of hybridization of the probe to the mRNA of a normal control (i.e., non-cancerous) cell from the same type of epithelial tissue, wherein an amount of hybridization to the mRNA of the test cell substantially less than the amount obtained with the mRNA of the normal control cell (preferably less than about one-half, more preferably less than about one-third, and more preferably less than about one-tenth the control amount of hybridization) is an indication that the test cell is cancerous. An absence of hybridization with the mRNA of the test cell is an indication that the test cell is from an advanced, probably metastatic tumor, while an amount of hybridization that is detectable but substantially less (e.g., one-third or'less) than that measured in a normal cell of the same tissue type is an indication that the test cell is from an early stage carcinoma that is probably not yet metastatic. The assay can be conveniently carried out using standard techniques of in situ hybridization or Northern analysis.

The antibody-based assays of the invention are comparable to the above. The proteins of the test cell, or from a fluid bathing the test cell, are contacted with an antibody (polyclonal or monoclonal) specific for maspin, and the amount of immunocomplex formed with such proteins is compared with the amount formed by the same antibody with the proteins of a normal control cell (or from a fluid bathing the normal control cell) from the same type of epithelial tissue as the test cell. An amount of immunocomplex observed with the proteins of the test cell substantially less than the amount observed with the proteins of the normal control cell (e.g., less than about one-half, preferably less than about one-third, and more preferably less than about one-tenth) is an indication that the test cell is cancerous. The absence of consistently detectable immunocomplex formed with the proteins of the test cell is an indication that the test cell is from an advanced, probably metastatic tumor, while an amount of immunocomplex formation that is consistently detectable but less (e.g., one-third or less) than that measured in a normal cell of the same tissue type is an indication that the test cell is from an early stage carcinoma that is probably not yet metastatic. (By consistently detectable is meant that, in all or nearly all of repeated trials, an amount greater than the applicable background level is observed.)

The immunoassay of the invention alternatively can be carried out on a biological fluid, since maspin protein is normally secreted by epithelial tissues such as mammary tissue. Such an assay would require obtaining a sample of a biological fluid (e.g., blood, serum, urine, saliva, milk, ductal fluid, tears, or semen) from an individual, which biological fluid would, in an individual free of carcinoma, contain a control amount of maspin. The sample, or protein derived from the sample, is contacted with the anti-maspin antibody, and the amount of immunocomplex formed is determined. This amount indicates the concentration or amount of maspin in the biological fluid. When compared to a sample previously or subsequently obtained from the same individual, this method provides a way to monitor the appearance, progress, or treatment of a carcinoma.

In another aspect, the invention features a method for screening candidate anticancer compounds, using as a screening tool cells (e.g., primary cells or an established cell line) from a carcinoma derived from a given tissue type in which the maspin gene is intact but down-regulated: that is, the level of expression of maspin in that carcinoma is significantly lower than (e.g., less than one-third of) the level of expression in normal epithelial cells from that type of tissue. The tissue may be from a human or another animal, and is preferably mammary epithelium. It is preferred that there be no detectable expression of maspin in the cells to be employed in the screen: i.e., the maspin gene is entirely shut down. The screening method includes the step of providing two samples of the screening cells, one of which is treated with a candidate anticancer compound and the other of which serves as control. The level of expression of maspin in the treated sample is compared with the level in the second sample, a higher level in the first sample being an indication that the candidate compound is a potential anticancer agent. The level of expression can be determined by use of hybridization methods or by immunoassay, as described herein.

As an alternative way of screening for potential anticancer agents, one can use any cell in which expression of maspin is undetectable, but which contains an intact maspin gene. This cell would be treated with a candidate anticancer compound, and a determination made of whether expression of maspin is thereby increased in the cell. Such an increase of maspin expression is an indication that the candidate compound is a potential anticancer agent. As above, the level of expression can be determined by use of hybridization methods or by immunoassay.

Also within the invention are methods of treating a carcinoma, where the carcinoma is one in which expression of maspin is decreased relative to normal cells of the tissue type from which the carcinoma cells were derived. In these methods, the patient is treated with an effective amount of a compound which increases the amount of maspin in, or in the immediate vicinity of, his or her carcinoma cells. This compound could be, for example, maspin or a biologically active fragment thereof; a nucleic acid encoding maspin and having expression control elements permitting expression in the carcinoma cells; or an agent which increases the level of expression of a maspin gene endogenous to the carcinoma cells (i.e., which up-regulates expression of the maspin gene).

The invention also features methods for *in vivo* screening of candidate anticancer agents, or for determining whether a particular carcinoma, in which maspin expression is down-regulated in comparison with normal cells of the same tissue type, is treatable with a given compound that increases expression of maspin. Such a method would include the steps of (1) introducing a carcinoma cell (e.g., a from a mammary carcinoma) into a severely immunodeficient animal (e.g. a nude mouse), the expression of maspin (SEQ ID NO:2) in the cell being down-regulated in comparison with that in a normal cell of the same type of tissue as the carcinoma cell; (2) treating the animal with a compound which increases the concentration of maspin in or around (i.e., in the immediate vicinity of) the carcinoma cell; and (3) determining whether this treatment affects the rate of proliferation or metastasis of the carcinoma cell in the animal, wherein a decrease in the rate of proliferation or metastasis in the presence of the compound is an indication that (a) the compound is potentially useful for treatment of carcinomas, and (b) the carcinoma from which the cell is derived is potentially treatable with the compound.

Besides the *in vivo* assay described above, one can also utilize an *in vitro* assay for carcinoma cell invasive capacity based upon the assay described in detail below. Such an assay would include the steps of (1) providing a first and a second carcinoma cell, which cells express maspin (SEQ ID NO:2) to a degree substantially lower than (i.e., less than one-third of, when measured by hybridization to cellular mRNA) that of a normal cell from the same type of tissue as said carcinoma cells; (2) treating the first cell with a compound which increases the concentration of maspin (SEQ ID NO:2) in or around the first cell; and (3) comparing the invasive capacity of each of the first and second cells in an *in vitro* assay such as that described below, wherein a decrease in invasive capacity of the (treated) first cell relative to that of the (untreated) second cell is an indication that (a) the compound is potentially useful for treatment of carcinomas, and (b) the carcinoma from which the cells are derived is potentially treatable with the compound. This assay is also useful for detecting maspin activity in a biological sample (e.g., during the process of purification of maspin, or for testing the biological activity of maspin fragments or derivatives, or for determining the presence of maspin in a sample of blood, milk, or other biological fluid), wherein a decrease in invasive capacity of the (treated) first cell relative to that of the (untreated) second cell is an indication that maspin, or maspin biological activity, is present in the sample.

Other features and advantages of the invention will be apparent from the following detailed description, and from the claims.

### Brief Description of the Drawings

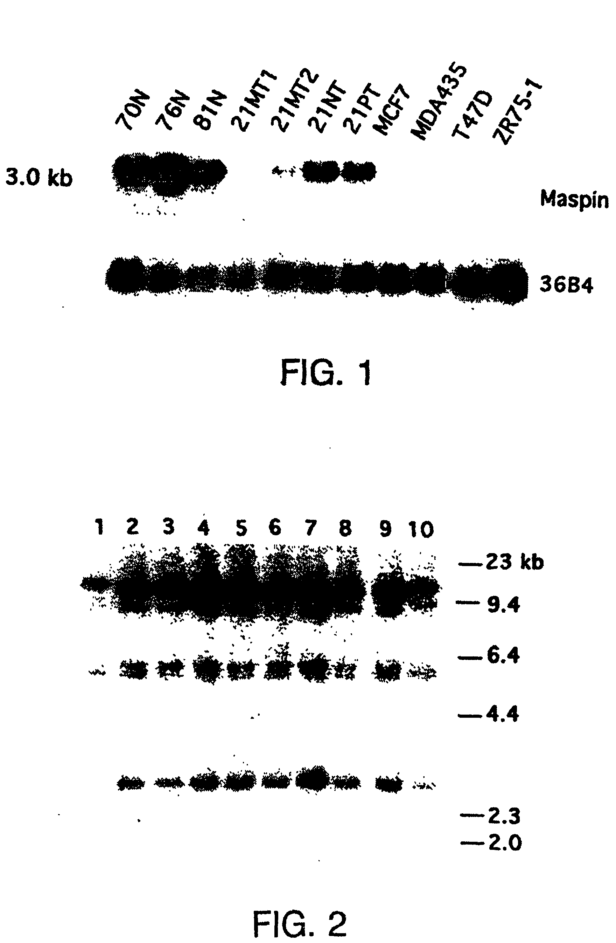

Fig. 1 is a Northern blot analysis of maspin expression in normal and tumor cells. Total cellular RNA was isolated from exponentially growing cells cultured in DFCI-1 medium (Band et al., Proc. Natl. Acad. Sci. USA 86:1249, 1989). 20 ug RNA was electrophoresed on 1% formaldehyde agarous gel, transferred to nylon membrane and hybridized with ³²P-labeled maspin probe. Lanes 1-3, normal breast epithelial cells 70N, 76N, 81N; lanes 4-11, breast tumor cells 21MT1, 21MT2, 21NT, 21PT, MCF7, MDA-MB-435, T47D, and ZR75-1.

Fig. 2 is a Southern blot analysis of the maspin gene. DNA (20mg) was digested with XbaI, fractionated on 1% agarose gel and transferred to nylon membrane. The blot was hybridized with ³²P-labeled full length maspin cDNA. Lanes 1-2,.normal breast cell 70N and 76N; lanes 3-10, breast tumor cell lines 21MT1, 21MT2, 21NT, 21PT MDA-MB-231, MDA-MB-435, MCF7, ZR75-1.

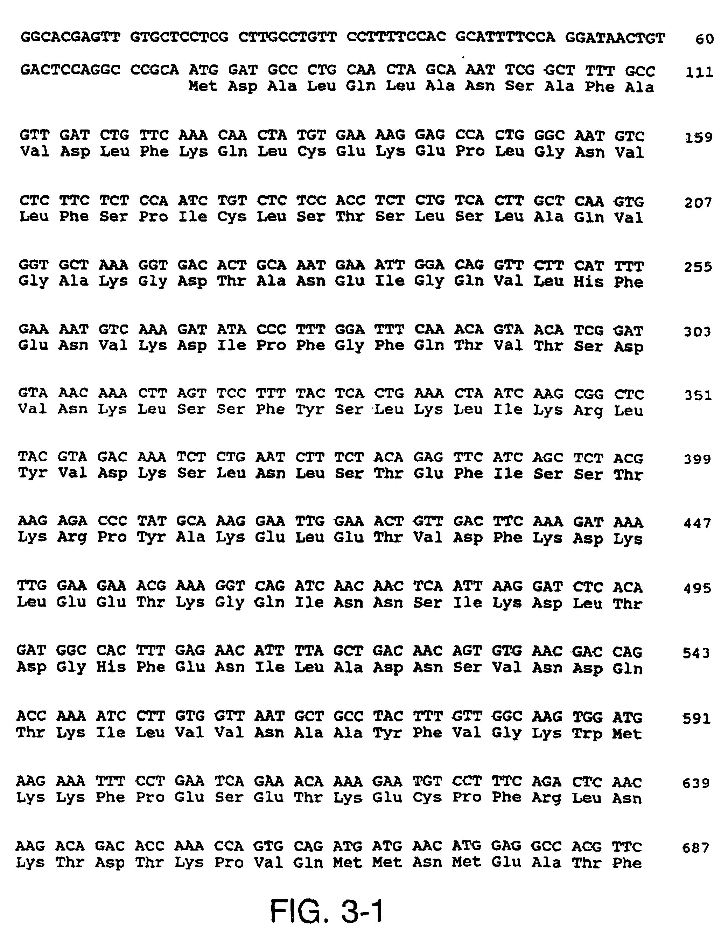

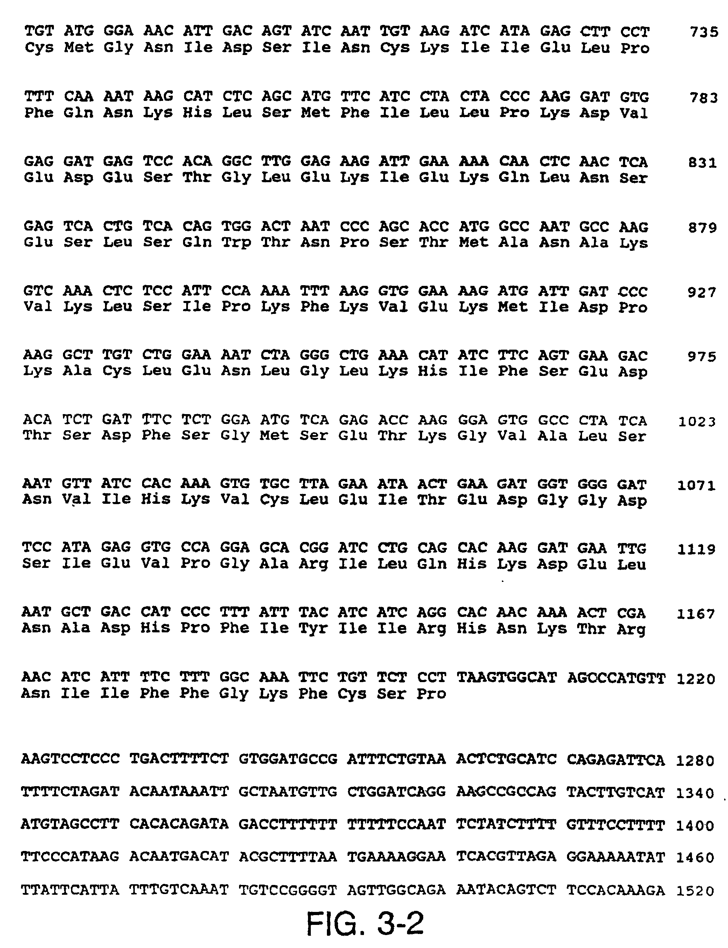

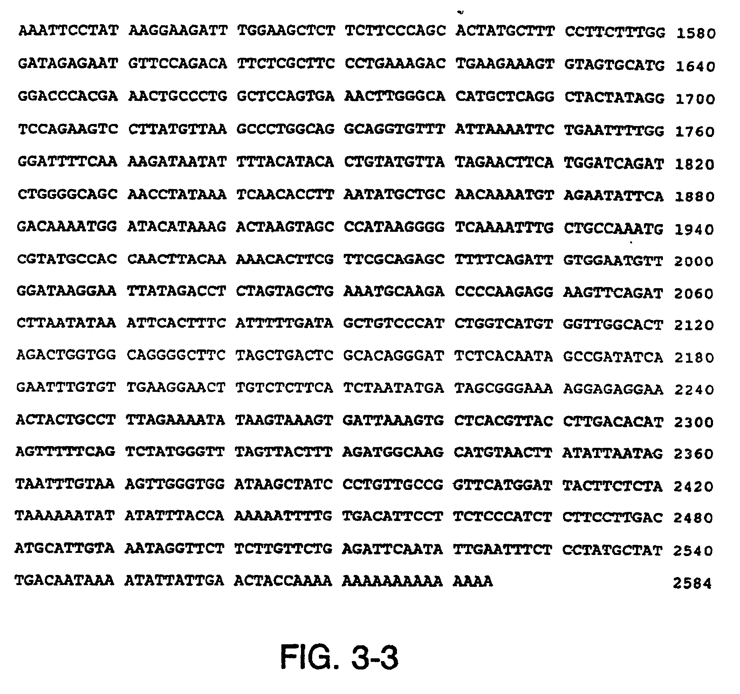

Fig. 3 is a representation of the complete cDNA and predicted amino acid sequence of maspin (SEQ ID NO:1). cDNA sequencing was performed using ABI 373A Automated DNA Sequencer at the core facility of Dana-Farber Cancer Institute. The polyadenylation signal is underlined.

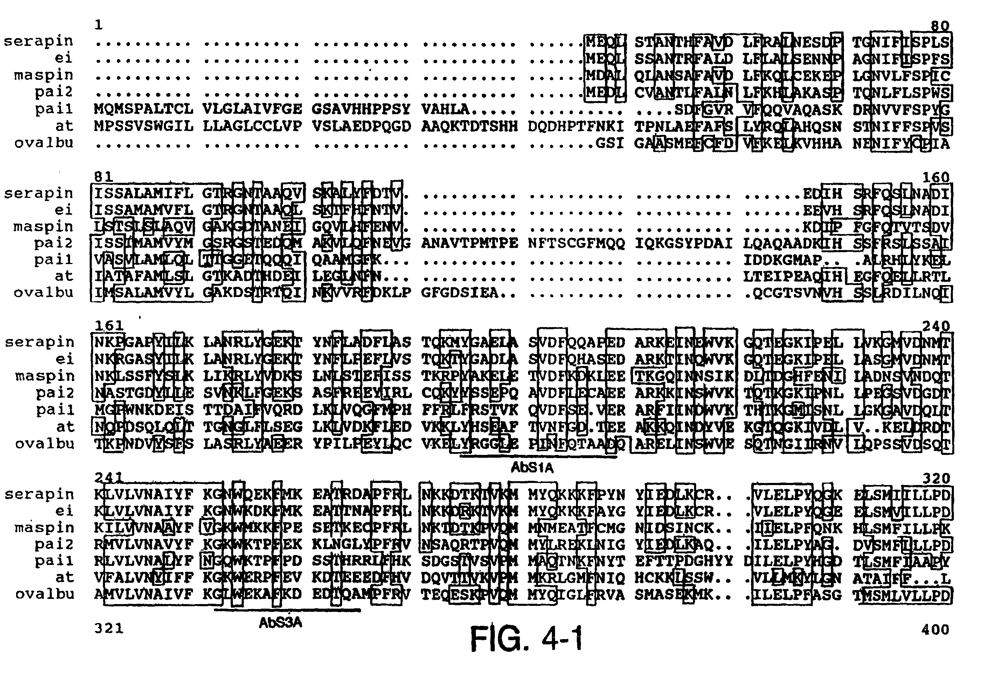

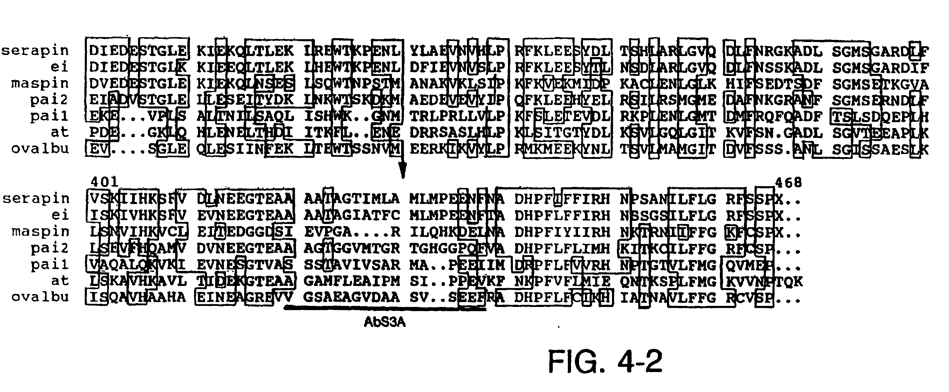

Fig. 4 is a comparison of the amino acid sequence of maspin (SEQ ID NO:2) with that of other serpins. Identical residues are boxed. Three regions used for antibody production are underlined. The arrow denotes the proposed reactive center of maspin. At, α1-antitrypsin (SEQ ID NO:3); ei, human monocyte/neutrophil elastase inhibitor (SEQ ID NO:4); ovalbu, ovalbumin (SEQ ID NO:5); pail, human plasminogen activator inhibitor type 1 (SEQ ID NO:6); pai2, human plasminogen activator type 2 (SEQ ID NO:7); serapin, horse serapin (SEQ ID NO:8).

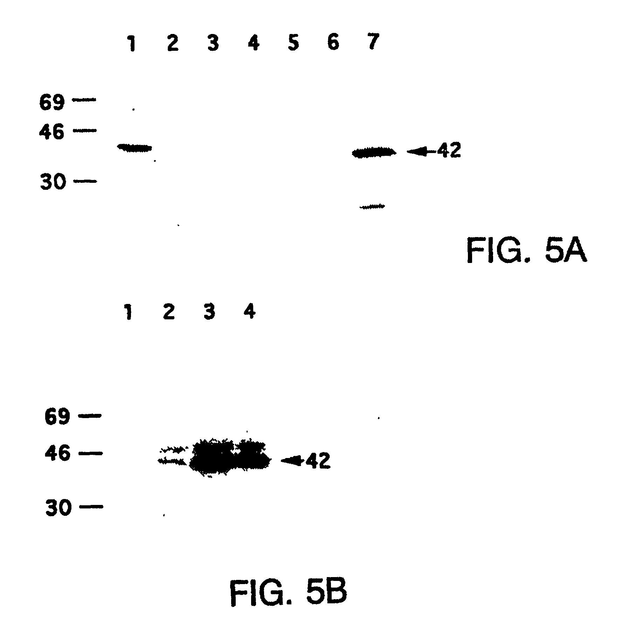

Fig. 5A is a Western blot analysis of maspin protein from normal and tumor cells. Cells were lysed in SDS-loading batter, and extracts were electrophoresed on 10% SDS gel and transferred on to Immobilon membrane. Maspin was detected by antiserum AbS1A using the ECL system. Lane 1, 76N; lanes 2-5, tumor cells MCF7, MDA-MB-435, ZR75-1; lane 6, MDA-MB-435 neo transfectant; lane 7, MDA-MB-435 maspin transfectant.

Fig. 5B is a Western blot showing detection of maspin protein in normal cells, using immunoprecipitation. Growing normal cells (70N) were labeled with an ³⁵S-labeled mixture of methionine and cysteine, and immunoprecipitated with on of four anti-maspin antibodies (lane 1, preimmune serum; lane 2, AbS3A; lane 3, AbS4A; lane 4, AbS1A).

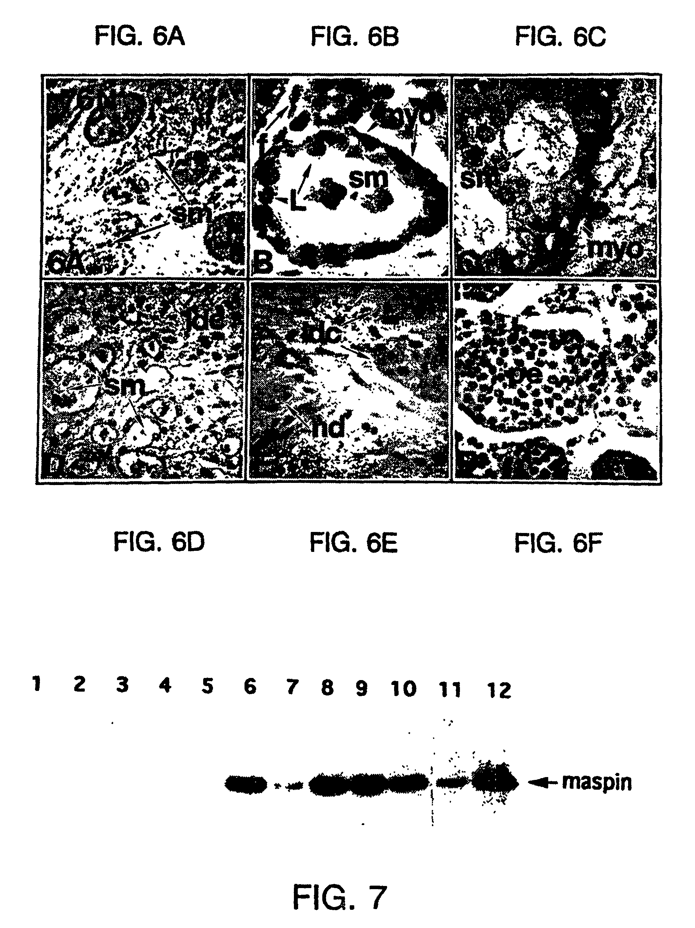

Figs. 6A-F are photographs of tissue sections stained by imrounoperoxidase to illustrate maspin protein expression in acetone-fixed normal mammary epithelial cell cultures (Fig. 6A), and in formalin-fixed paraffin embedded sections of benign (Fig. 6B) and carcinomatous breast tissue (Fig. 6C, ductal carcinoma in situ; Figs. 6D and 6E, invasive ductal carcinomas; Fig. 6F, pleural effusion containing metastatic breast cancer). Maspin-immunoreactive sites were unmasked in formalin-fixed sections by pretreatment of the sections in 10% sucrose at 80°C for 2 hours. Both cell cultures and tissue sections were incubated with 5 µg/ml of AbS4A followed by immunoperoxidase detection employing biotinylated tyramine (Adams, J. Histochem. Cytochem 40:1457, 1992). 3-Amino-9-ethylcarbazole was used as the chromagen and nuclei were counterstained with Mayer's hematoxylin. Presorbtion of the primary antibody AbS4A with immunizing peptide eliminated all specific maspin staining. Key: sm, secreted maspin; L, luminal cell; myo, myoepithelial cell; f, fibroblast; dcis, ductal carcinoma in situ; idc, invasive ductal carcinoma; nd, normal benign breast duct; pe, pleural effusion.

Fig. 7 is a Western analysis of maspin in MDA-MB-435 transfectants. Maspin was detected by peptide affinity purified antibody AbS1A. Lanes 1-5, neo transfected; lanes 6-12, maspin transfectant clones T1, T4, T5, T6, T7, T2 and T3, respectively.

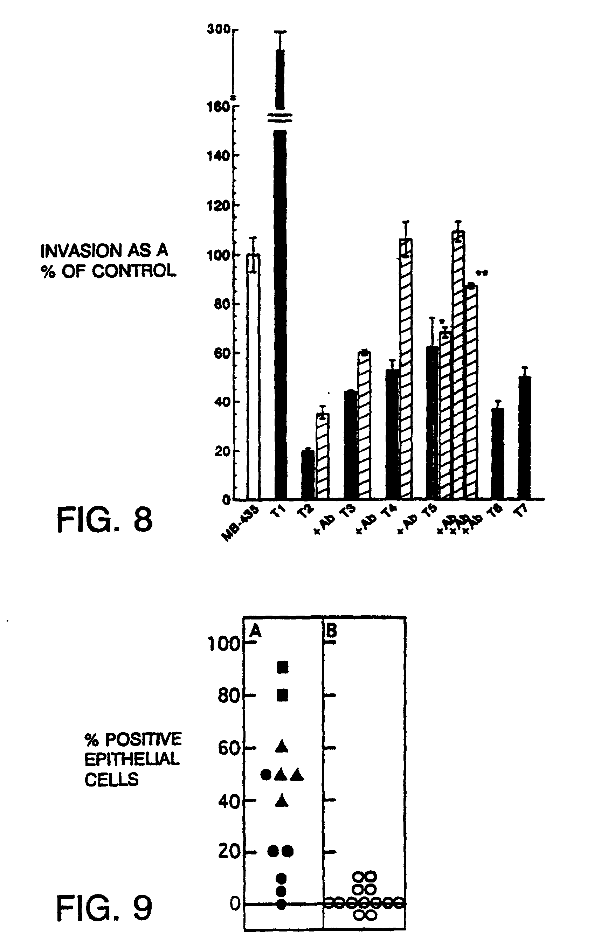

Fig. 8 is a bar graph illustrating the effect of maspin transfection into MDA-MB-435 cells on invasive potential *in vitro* in the presence or absence of antibodies to maspin. The invasive ability of maspin-transfected clones (T1-T7) to penetrate reconstituted basement membrane-coated (Matrigel; Becton Dickinson, Boston, MA) polycarbonate filters (containing 10mm pores) was measured over 72h using the Membrane Invasion Culture System (MICS). 1x10⁵ cells were seeded into the upper wells of the MICS chamber onto the Matrigel-coated filter in DMEM medium containing 10% NuSerum (Becton Dickinson). After 72h incubation at 37°C with constant O₂ and CO₂ exchange, the cells that invaded the filter were collected, stained and counted with the aid of a light microscope. The invasion data of the non-transfected MDA-MB-435 cells were normalized to 100%, and the invasion data of the experimental and control transfectants are expressed as a percentage of this control. The data represent the average of two separate experiments; error bars represent the standard error of the mean and are based on n = 6 for each experiment. To neutralize the activity of secreted maspin, selected clones were pretreated with AbS4A maspin antibody continuously during the course of the invasion assay at a concentration of 1.0mg/ml, unless stated otherwise. In selected experiments, additional concentrations of the antibody were tested: *0.1 and **3.0 mg.ml. The invasive potential of the untreated clones was normalized to 100%, and the invasive potential of the treated clones is indicated as a percentage of the untreated respective clones.

Fig. 9 is a chart illustrating the reactivity of AbS4A antiserum with several mammary carcinoma samples. Affinity-purified AbS4A (at 5 µg/ml) was reacted with formalin-fixed 5µm paraffin-embedded sections that were pretreated in 10% sucrose at 80°C for 1 hour. Antibody-antigen complexes were visualized by the modified immunoperoxidase method using biotinylated tyramine (Adams, J. Histochem. Cytochem. 40:1457, 1992). The % positive cells denotes the number of carcinoma cells that were reactive with AbS4A divided by the total number of tumor cells X 100 in (A) primary breast carcinomas, and (B) mammary lymph node metastases and pleural effusions (o). Each symbol represents a specimen from a different individual. Many tumor cells in ductal carcinomas in situ (■) expressed maspin. Differentiated components of invasive breast carcinomas (▲) expressed some maspin. Poorly differentiated neoplasms (•) failed to exhibit maspin immunoreactivity.

### Detailed Description

### IDENTIFICATION OF MASPIN

Using subtractive hybridization (as described in U.S.S.N. 07/844,296), a new member of the serpin family has been isolated, cloned, sequenced, and partially characterized. The gene was named maspin because of its sequence similarity to other serpins, and its initial identification in mammary epithelial cells. As shown in Fig. 1, the maspin gene expresses a single 3.0 kb mRNA in three normal mammary epithelial cell strains (Band et al., Proc. Natl. Acad. Sci. USA 86:1249, 1989) but not in a series of tumor cell lines including those shown in Fig. 1 as well as MDA-MB-157, MDA-MB-231, MDA-MB-436, MDA-MB-468, BT549, and HS578T (not shown). Two cell lines from primary tumors of a single patient (21PT and 21NT) (Band et al., Cancer Res. 50:7351, 1989) expressed maspin mRNA, but at a much reduced level compared with the normal cells. Neither foreskin fibroblasts nor breast-derived fibroblasts expressed detectable maspin mRNA. Southern analysis of DNA from normal and tumor cells (Fig. 2) using the restriction enzyme XbaI, which produced 5 fragments that hybridized with the maspin probe, showed no differences in pattern among them. Thus the gene is present and unaltered at this level of resolution in the tumor cell lines including the 21T series, in which the primaries (21PT, 21NT) express the mRNA and the cells of metastatic origin do not. This evidence suggests that maspin is a Class II candidate tumor suppressor gene, down-regulated but not mutated in cancer cells (Lee et al., Proc. Natl. Acad. Sci. USA 88:2825, 1991; Sager et al., FASEB J. 7:964-970, 1993)

Maspin cDNA (SEQ ID NO:1) was isolated from a normal human mammary epithelial cell library (76N), as described in U.S.S.N. 07/844,296. The cDNA sequence contains 2584 nucleotides with a polyadenylation signal located 16 nucleotides from the 3' terminus of the sequence, as shown in Fig. 3. The full length sequence includes 75 nucleotides of the 5' untranslated region and 1381 nucleotides of 3' untranslated region. The initiation codon and surrounding nucleotides fit the Kozak consensus (Kozak, Nucleic Acid Res. 12:857, 1984). The cDNA encodes a protein of 375 amino acids with an N-terminal methionine and C-terminal valine. Maspin also contains 8 cysteine residues, and may utilize two or more disulfide bonds to stabilize its tertiary structure.

Multiple alignment studies based on data base searches using BLAST at the National Center for Biotechnology Information and analyzed by the GCG Pileup program demonstrate close homology to the serpin superfamily of serine proteinase inhibitors (see Fig. 4). Serpins are a diverse family of proteins related by virtue of primary sequence homology spanning the entire length of each molecule, and varying from 15-50% at the amino acid level and higher at the DNA level. Maspin exhibits closest homology at the protein sequence level to the equine (43%) and human neutrophil-monocyte elastase inhibitors (39%), human PAI-2 (31%), human squamous cell carcinoma antigen (SCCA, 34%), and chicken egg albumin (31%).

### THE SERPIN FAMILY

Serpin molecules possess important physiological functions, including proteinase inhibition (inhibitors of complement activation, coagulation, kinin formation, and fibrinolysis), hormone transport (thyroxine binding globulin, cortisol binding globulin), vasoactive peptide donors (angiotensinogen), and unknown function (ovalbumin) (for reviews see Travis et al., Biol. Chem Hoppe-Seyler 371:3, 1990; Huber et al., Biochem. 28:1, 1989).

The crystallographic structures have been solved for native and cleaved ovalbumin, cleaved α1-antitrypsin, cleaved α1-antichymotrypsin, and latent plasminogen activator inhibitor-1 (Stein et al., Nature 347:90, 1990; Wright et al., J. Mol. Biol. 213:513, 1990; Loebermann et al., J. Mol. Biol. 177:531, 1984; Baumann et al., J. Mol. Biol. 218:595, 1991; Mottonen et al., Nature 355:270, 1992). In each case, the structures have proven to be' very similar, indicating a conserved molecular framework. These studies confirm the usefulness of molecular modeling to make predictions concerning the unsolved structures of other serpins.

Active inhibitory serpins (S-form) interact with their target proteases with a 1:1 stoichiometry to form stable, denaturation-resistant complexes, in which the protease is inactive. Of primary importance in determining the specificity of the target protease is the nature of the p₁ residue of the reactive center. Serpins with Ala, Val, or Met at the p₁ position are inhibitors of elastase-like proteinases, while serpins with Arg at the p₁ position inhibit trypsin-like proteases.

The alignment of maspin with other serpins (Fig. 4) provides preliminary evidence that maspin may also function as a proteinase inhibitor. The homology alignment identifies Arg as the putative p₁ residue in maspin, suggesting that it may inhibit trypsin-like proteases such as plasmin, uPA, and tPA. Because of the gap preceding the reactive site peptide bond, other alignments are possible, but each likely alignment provides a p₁ residue with the potential for generating inhibitory activity.

### IDENTIFICATION OF MASPIN PROTEIN USING ANTI-MASPIN ANTIBODIES

Three poorly conserved sequences (underlined in Fig. 4 as S1A, S3A, and S4A) were selected as the basis for designing synthetic oligopeptides for polyclonal antibody production, using conjugation to keyhole limpet hemocyanin [Harlow et al., Antibodies, A Laboratory Manual, Cold Spring Harbor Laboratory, New York, 1988 (Chapter 8)]. The antisera were respectively designated as AbS1A, AbS3A and AbS4A. AbS4A recognizes the reactive center loop encompassing the putative p1-p1' residue. As shown in Fig. 5A, a 42 kDa band was detected on a reducing SDS gel, both in normal cells (70N; lane 1) and in tumor cells (MDA-MB-435) transfected with maspin cDNA (lane 7). This molecular weight is consistent with the estimated size based on the primary sequence. All three antibody preparations reacted with this 42 kDa protein. No protein was detected in breast tumor cell lines MCF7, MDA-MB-468, MDA-MB-435, and ZR75-1, or in MDA-MB-435 transfected with control vector (lanes 2-6). All three antisera precipitated a 42 kDa band from normal cell extracts (Fig. 5B). These results demonstrate that the maspin gene encodes a 42 kDa protein present in normal mammary epithelial cells and absent in tumor cell lines that do not express the mRNA.

### MASPIN EXPRESSION IN BENIGN AND MALIGNANT BREAST

Indirect immunofluorescence microscopy of a normal human mammary epithelial cell strain (76N) demonstrated that maspin protein is localized mainly to the pericellular space, with weak staining in the cytoplasm (Fig. 6A). These results demonstrate that maspin is secreted into the ECM, and may interact with its target protease in the ECM and/or on the plasma membrane. Primary mammary tumor cells grown in culture (21PT) exhibited weak staining with a pattern similar to the normal cells, consistent with their low-level expression of maspin mRNA (Fig. 1), whereas MDA-MB-435 cells were negative. Each of the maspin antisera AbS1A, AbS3A, and AbS4A generated similar staining patterns that could be preabsorbed by the corresponding immunizing peptide.

As shown in Figs. 6A and B, acetone-fixed cryosections and formalin-fixed, paraffin-embedded sections of benign breast tissues (n=6) and benign epithelium adjacent to invasive carcinomas were maspin positive when immunostained with AbS4A. Maspin expression was particularly intense in myoepithelial cells, both within large ducts and terminal duct lobular units (TDLU). Luminal epithelial cells were heterogeneously positive (often showing weak granular cytoplasmic immunopositivity of some cells), with more intense apical reactivity and some positivity of intraluminal secreted material (Fig. 6B). Inflammatory and stromal cells were always negative.

Twelve invasive carcinomas of the breast, eleven regional lymph node metastases, two pleural effusions containing metastatic breast cancer, and adjacent *in situ* epithelial elements were also evaluated for maspin expression (Fig. 9). Carcinoma *in situ* was weakly immunopositive, and apical expression was occasionally noted. Maspin expression was highest within myoepithelial cells (adjacent to the basement membrane). Secreted maspin was sometimes observed in the luminal space of benign breast (Fig. 6B) and ductal carcinomas *in situ* (Fig. 6C), and rarely in invasive ductal carcinomas, as for example in the well-differentiated tubular variant (Fig. 6D). Most malignant cells in invasive carcinomas failed to express maspin (Fig. 6E), but a minority of cells in well differentiated tumors expressed maspin focally (Fig. 9). Maspin was undetectable or very weakly expressed in all lymph node metastases and pleural effusions examined (Fig. 6F). These findings suggest a biological role for maspin in the benign breast and a potentially pivotal alteration in maspin expression during the progression of breast cancer.

### DECREASED GROWTH IN NUDE MICE OF TUMOR TRANSFECTANTS EXPRESSING MASPIN

Tumor cell line MDA-MB-435 forms tumors at the site of orthotopic injection and metastasizes in nude mice (Price et al., Cancer Res. 50:717, 1990). To investigate whether maspin has inhibitory effects on tumor formation in nude mice, MDA-MB-435 cells were transfected with an expression vector encoding maspin under the control of the CMV promoter (Tomasetto et al., J. Cell Biol. 122:157, 1993). The exogenous gene expressed a 3.0 kb mRNA and a 42 kDa protein at levels similar to those seen in normal cells, whereas no maspin was expressed by the neo-controls (Fig. 7). The low levels of maspin in transfected clones T2 and T4 resulted from instability in maspin transfectants. In cell culture, the maspin transfectants, the neo-controls, and the MDA-MB-435 parental cells all grew at the same rate in alpha-MEM medium containing 5% fetal calf serum (data not shown).

Four maspin transfectants and two vector control transfectants were tested in nude mice as described (Price et al., Cancer Res. 50:717, 1990). Table 1 summarizes the results obtained from two duplicate experiments. At 10 weeks post-inoculation, all mice were sacrificed, and their tumors excised and weighed. Between 6 and 10 weeks, some mice died due to tumor burden or illness. These mice are not included in Table 1. Three of the four maspin transfectant clones produced much smaller tumors than the vector control clones, whereas one clone (T1) grew at the same rate as MDA-MB-435 and the neo-controls. Using the Student t-test, the differences between maspin transfectants and neo-controls were significant whether or not T1 was included in the calculations.

These results show unequivocally that maspin expression leads to growth inhibition of injected transfectants compared with controls. It is obvious by inspection that T1 is not inhibited, whereas the other three transfectants are strongly inhibited. The inhibitory effect of maspin on tumor growth is not unexpected, since other laboratories have reported effects of proteases in inducing growth factor expression indirectly, perhaps via cleaved components of the ECM (Testa et al., Cancer Metastasis Rev. 9:353, 1990).

### DECREASED INVASIVE CAPACITY OF MASPIN TRANSFECTANTS

An *in vitro* assay of tumor cell invasion through reconstituted basement membrane matrix (Matrigel) has been used to assess the functional activity of maspin (Hendrix et al., Cancer Letters 38:137, 1987). Seven maspin transfectant clones and 5 neo vector transfectant clones were compared with the parental MDA-MB-435 cells. Six (T2-T7) of the 7 maspin transfectant clones showed reduced invasive ability; as shown in Fig. 8, this difference was neutralized in a dose-dependent manner with the peptide affinity-purified antibody AbS4A. The antibody blocked the inhibitory effect of the recombinant maspin produced by the transfected cells, resulting in elevated invasive activity. Since AbS4A recognizes the reactive center of the protein, it is likely that the site of interaction with the target protease was blocked.

By immunofluorescence microscopy, we noted that maspin expression was heterogeneous in the pre-invasion cells. However, in the post-invasion cells, staining revealed that more than 95% were maspin-negative. Thus, the effectiveness of maspin in inhibiting invasion is somewhat underestimated in these experiments, owing to heterogeneity of expression in the transfectant population. Furthermore, our evidence that only the maspin-negative cells crossed the Matrigel barrier further demonstrates that the cells expressing maspin were inhibited in their ability to invade.

In addition, five neo-control transfectants were tested for their invasive capacity (data not shown). Of these, two expressed invasive activity comparable to the parental cells, whereas three of them showed a decrease in invasive activity. However, none of the five neo-controls responded to the AbS4A antibody, indicating that maspin was not responsible for the decreased invasiveness of the controls. This result is consistent with the absence of maspin protein in the neo-controls, as shown in Fig. 5A. In light of Liotta's three-step invasion model (Liotta et al., Cancer Res. 51:5054, 1991) (i.e., adhesion, degradation, and motility), the adhesive ability of all 12 transfectants (7 containing maspin and 5 controls) to Matrigel matrix were examined; no differences among them were found (data not shown).

These data support the hypothesis that the activity of maspin is associated with the inhibition of tumor cell invasive potential. It is noteworthy that the same transfectant clone (T1) which showed no inhibition of invasive potential in the invasion assay, also showed no decrease of tumor size when tested in'the nude mouse assay. This clone, which is more invasive than the parental tumor cells, may overexpress a novel protease, and merits further investigation.

### CHROMOSOMAL LOCATION OF MASPIN

A panel of 24 human-rodent somatic cell hybrids was used to map the chromosomal location of the maspin gene. All hybrids retained a single human chromosome except one line that contained both chromosome 20 and a low percent of chromosome 4, and one that contained both chromosomes 1 and X. In human DNA, the maspin probe detected a major 5.4 kb HindIII fragment, which was clearly resolved from a weakly hybridizing Chinese hamster fragment of about 20 kb. The presence of the 5.4 kb human maspin sequence in the hybrid clones correlated only with the presence of human chromosome 18 (Table 2). Only one of the 24 hybrids analyzed was positive for maspin; this hybrid contained chromosome 18 as the sole human DNA. No discordancies for localization to chromosome 18 were found, whereas there were at least two discordancies for localization to any other chromosome. The maspin gene has been localized to 18q21.3, the same chromosomal region as a closely related gene, PLANH2, that encodes plasminogen activator inhibitor-2 (PAI-2) (LeBeau et al., Human Gene Mapping 11, cytogenet. Cell Genet. 58:739, 1991).

### Example 1: Recombinant maspin

Using the information provided above, one of ordinary skill can generate a synthetic DNA probe consisting of a 20-nucleotide segment of the maspin cDNA sequence (SEQ ID NO:1), and use that probe to screen at high stringency a cDNA library from an appropriate epithelial cell line such as MCF7. Alternatively, one could design two appropriate PCR primers, based upon the disclosed cDNA sequence, and generate a maspin cDNA either from the same library, or directly from the mRNA of that cell line. Both of these procedures are standard ones readily carried out by one of ordinary skill in the art. Sequencing of the cDNA so obtained will confirm that it is the maspin cDNA disclosed herein. Multiple copies of the CDNA are readily produced by inserting the cDNA into a recombinant vector, and using that vector to transfect a prokaryotic host such as *E. coli.* This cDNA, or a fragment thereof, can be used to screen epithelial cell cDNA libraries from species other than human [e.g., mammalian species such as mouse, rat, guinea pig, hamster, rabbit, cow, pig, horse, sheep, monkey, and ape; or non-mammalian animals such as birds or insects (e.g., Drosophila); or microorganisms such as yeast] in order to identify the maspin homologs in such other species. It is likely that the stringency of the hybridization conditions would have to be adjusted to take into account the probable lack of complete sequence identity with the human cDNA.

Once the desired maspin cDNA is in hand, it can be inserted into an expression vector and used in an appropriate expression system to generate recombinant maspin protein. The expression system can be any standard system, including prokaryotic (e.g., E.coli), eukaryotic (e.g., yeast, CHO cells, COS cells, or baculovirus in insect cells), or cell-free. Since the protein appears to be secreted, it can be collected from the culture filtrate of E. coli, or from the medium bathing the transfected insect or other eukaryotic cells. Standard methods of protein purification, optionally including passage over an immunoaffinity column, can be employed to isolate the recombinant protein.

### Example 2: Diagnostic assay utilizing hybridization probe

As described above, a nucleic acid probe containing some or all of the maspin-encoding sequence of the invention (SEQ ID NO:1) can be used to detect maspin mRNA in a sample of epithelial cells (e.g., a tissue section) suspected of being cancerous. The probe used would be a single-stranded DNA or RNA (preferably DNA) antisense to the coding sequence shown in Fig. 3. It coula be produced by synthetic or recombinant DNA methods, and labelled with a radioactive tracer or other standard detecting means. The probe could include from 15 to the full 1125 nucleotides of coding sequence, and preferably is at least 30 nucleotides long. The assay can be carried out by standard methods of *in situ* hybridization or Northern analysis, using stringent hybridization conditions. Control hybridization assays can be run in parallel using normal epithelial cells or tissue sections from the same type of tissue as the test sample, and/or cells from a known carcinoma or carcinoma-derived cell line, or a cancer-containing tissue section. Cells which exhibit a substantially decreased level, or absence, of hybridization to the probe, compared to the .level seen with normal epithelial cells, are likely to be cancerous. The amount of hybridization can be quantitated by standard methods, such as counting the grains of radioactivity-exposed emulsion on an *in situ* hybridization assay of a biopsy slide, or by densitometric scan of a Northern blot X-ray film. Alternatively, comparison of the test assay results with the results of the control assays can be relative rather than quantitative, particularly where the difference in levels of hybridization is dramatic. This assay is useful for detecting cancerous cells in breast epithelial tissue or in any other type of tissue in which maspin is normally expressed.

### Example 3: Diagnostic assay utilizing antibody probe

Antibodies specific for maspin can be generated by standard polyclonal or monoclonal methods, using as immunogen a purified, naturally-occurring maspin; recombinant maspin; or any antigenic fragment of maspin which induces antibodies that react with naturally-occurring maspin. The latter fragment can be produced by synthetic or recombinant methods, or by proteolytic digestion of holo maspin. (Three examples of fragments useful for antibody production are described above.) If desired, the antigenic fragment can be linked by standard methods to a molecule which increases the immunogenicity of the fragment, such as keyhole limpet hemocyanin (as described above). The polyclonal or monoclonal antibodies so produced can be screened using purified recombinant or naturally occurring maspin, or as described above, to select those which form an immunocomplex with maspin specifically.

The antibodies so produced are employed in diagnostic methods for detecting cells, tissues, or biological fluids in which the presence of maspin is decreased relative to normal cells, an indication that the patient has a carcinoma. The sample tested may be a fixed section of a tissue biopsy, a preparation of cells obtained from a suspect tissue, or a sample of biological fluid, such as blood, serum, urine, sweat, tears, cerebrospinal fluid, milk, ductal fluid, or semen. Standard methods of immunoassay may be used, including those described above as well as sandwich ELISA. If the tested cells express no detectable maspin protein in this assay, while normal cells of the same tissue type do express a detectable level of maspin, the tested cells are likely to represent an advanced, metastatic carcinoma. If the tested cells express a decreased but consistently detectable level of maspin, the tested cells are probably from an early stage carcinoma that is not yet metastatic. Where the sample tested is a biological fluid into which maspin would normally be secreted, the fluid may be directly contacted with the anti-maspin antibody, or can be first partially processed (e.g., centrifuged, pre-cleared with other antibodies, dialyzed, or passed over a column) before using the anti-maspin antibody. The amount of immunocomplex formed between the proteins of the sample and the anti-maspin antibody is then determined, and can be compared to a normal control run in parallel, or to a previously-determined standard.

### Example 4: In vivo and in vitro assays

The *in vivo* assay described above, in which tumor growth is measured in severely immunodeficient mice (e.g., nude mice), is useful in a number of applications concerning the present invention. For example, the assay can be used to determine (1) whether or not growth of a given carcinoma is inhibited by treatment either with maspin or an agent which increases the concentration of maspin in the carcinoma cells; or (2) whether or not a given candidate compound, which may be known to increase maspin expression in carcinomas in which maspin expression is down-regulated, can in fact inhibit growth of such carcinomas. The nude mice (or any other severely immunodeficient animal, such as a rat, rabbit, or other mammal) can also be adapted to study the effect of a given treatment on the rate of metastasis of the tumor, using standard methods of *in vivo* analysis of metastasis. A second type of assay described above, the *in vitro* assay of tumor cell invasion through reconstituted basement membrane matrix (e.g., MATRIGEL®), is also generally useful with respect to the present invention. Using this assay, the increase in invasive capacity of a given carcinoma over time, or of a series of carcinomas from different patients, can be correlated with the degree of inhibition of maspin expression in each carcinoma sample. The assay can be used to screen various treatment protocols to determine whether a given maspin-increasing protocol is effective in reducing invasive capacity in a given carcinoma.

### Example 5: Assay for presence of intact gene

If expression of the maspin tumor suppressor gene is down-regulated in the cells of a given carcinoma, but the gene remains present and intact in such cells, it is possible that the cells could be treated in a way that stimulates re-expression of the gene and thereby reverses or at least halts the progression of the carcinoma. This strategy would require affirmation that the gene remains intact and therefore available for up-regulation in the particular cancer cells to be treated. A Southern analysis of genomic DNA from the cancer cells and normal cells, such as described above, would provide evidence that the maspin gene in the cancer cells is largely intact. Use of a battery of restriction enzymes would permit a more rigorous analysis of whether changes in the gene sequence had occurred in the cancer cells. One could use as hybridization probe full-length maspin cDNA (SEQ ID NO:1), maspin genomic DNA, or a fragment of either. To obtain maspin genomic DNA, a human genomic DNA library is probed with maspin cDNA (SEQ ID NO:1), using standard techniques such as described in Sambrook et al., Molecular Cloning: A Laboratory Manual (2nd Edition), Cold spring Harbor Laboratory, Cold Spring Harbor, NY (1989). The expression control elements of the naturally-occurring maspin gene (e.g., promoters and enhancers usually located 5' to the transcription start site, or within one or more introns, but also possibly in the 3' untranslated region) are of particular interest, since down-regulation of transcription is associated with tumor progression.

### Example 6: Screen for and use of therapeutic agents

Carcinoma or other cells in which the endogenous maspin gene is present but down-regulated can be used as a screening tool to identify compounds or treatment strategies which induce re-expression of the maspin gene. Re-expression of other down-regulated candidate tumor suppressor genes has been described: connexin 26, encoding a gap junction protein, by PMA (Lee et al., J. Cell Biol. 118:1213, 1992), and a small calcium binding protein, CaN19, by deoxyazacytidine (Lee et al., Proc. Natl. Acad. Sci. USA 89:2504, 1992). Of particular use in such a screen would be cell lines derived from an appropriate carcinoma, with a control being the same cells transfected with a vector encoding maspin cDNA linked to expression control elements which permit constitutive expression of the cDNA (e.g., the CMV promoter), as described above. However, other cell types with intact but unexpressed maspin genes would also be potentially useful in this screening assay. The cells would be treated *in vitro* with the candidate compounds, and the amount of maspin expression determined using either a hybridization assay (e.g., Northern analysis) or an immunoassay. The latter could be designed to detect intracellular maspin or secreted maspin, or both. If a given compound is found to stimulate maspin expression in the carcinoma cells, it could then be further tested to see whether treatment with the compound prevents carcinoma growth in the nude mouse model described above. A compound effective both in stimulating maspin expression and in preventing carcinoma growth is a potential therapeutic useful for the treatment of carcinomas down-regulated in maspin expression. Further evaluation of the clinical usefulness of such a compound would follow standard methods of evaluating toxicity and clinical effectiveness of anticancer agents.

### Example 7: Treatment with maspin

As discussed above, increasing the amount of maspin in a carcinoma cell appears to correlate with a decrease in both growth rate and invasive activity of the tumor. Thus, it is expected that treating a patient with maspin, or a biologically active (i.e., protease-inhibiting) fragment of maspin, will help counter the effects of down-regulation of the maspin gene in'the patient's carcinoma cells. Since maspin is a secreted protein, it is likely that it exerts its tumor growth-suppressing effect extracellularly. A useful treatment protocol will therefore be a simple method such as intravenous injection of the protein in a pharmaceutically acceptable solution in a dosage of 0.001 to 100 mg/kg/day, with the most beneficial range to be determined using routine pharmacological methods. This protocol has the advantage of potentially reaching all metastases of the tumor. Alternative routes of delivery would also be acceptable, such as intramuscular or subcutaneous injection, injection directly into the tumor site, or implantation of a device containing a slow-release formulation. If it is desired to ensure that the exogenous maspin protein is incorporated into the carcinoma cells themselves, the protein could be incorporated into liposomes or another form of carrier which permits substantial amounts of the protein to pass through the cell membrane. Liposomes would also help protect the protein from proteolytic degradation while in the bloodstream.

### Example 8: Genetic therapy

As disclosed above, an expression vector encoding maspin can be introduced into carcinoma cells, thereby increasing the production of maspin in the transfected cells, and decreasing the *in vivo* growth rate of tumors derived from these cells. The transfected cells are also shown above to have a decreased invasive character, compared to untransfected controls. This evidence indicates that the maspin DNA of the invention will be useful for genetic therapy to help control carcinomas characterized by down-regulated maspin expression, or to ensure that early-stage carcinomas which have not yet lost the ability to manufacture maspin do not progress through the progressively down-regulated stages. Standard methods of gene therapy may be employed: e.g., as described in Friedmann, *Therapy for Genetic Disease, T.* Friedman (ed.), Oxford Univ. Press, 1991, pp.105-121. Virus or plasmids containing a copy of the maspin cDNA linked to expression control sequences which permit expression in the carcinoma cell would be introduced into the patient, either locally at the site of the tumor or systemically (in order to reach any tumor cells that may have metastasized to other sites). If the transfected DNA encoding maspin is not stably incorporated into the genome of each of the targeted carcinoma cells, the treatment may have to be repeated periodically.

**TABLE 1.**

| | | |
|---|---|---|
| Tumor growth of maspin transfected MDA-MB-435 cells. Cells were resuspended in PBS, 5X10⁵ cells were injected into mammary fat pad of nude mice (8-10 weeks old for the first experiment, 4-6 weeks old for the second experiment). Each mouse was injected at two sites. Tumor development was monitored weekly. Numbers in parentheses are the numbers of tumors at 10 weeks. | | |

| Cells | Tumor/Site (6 weeks) | Mean Weight (gram) (10 weeks) |
|---|---|---|
| pCMVneo N1 | 8/10 | 0.74 (7) |
| pCMVneo N2 | 10/10 | 1.77 (6) |
| pCMVmaspin T1 | 8/10 | 1.67 (4) |
| pCMVmaspin T4 | 6/10 | 0.31 (7) |
| pCMVmaspin T5 | 5/10 | 0.35 (7) |
| pCMVmaspin T6 | 8/10 | 0.43 (9) |
| p = 0.034 (T1-T6) | | |
| P = 0.00057 (T4-T5) | | |

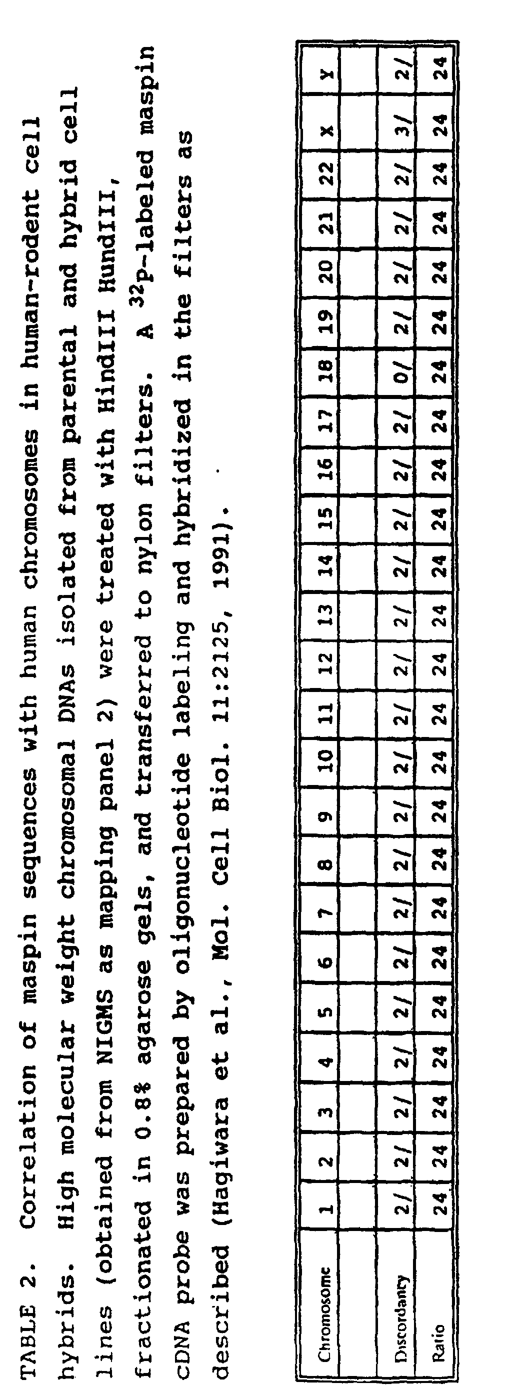

## Claims

1. A single or double stranded isolated DNA encoding a polypeptide having at least 90% sequence identity to maspin (SEQ ID NO:2).

2. The isolated DNA of claim 1, wherein said DNA comprises a sequence which hybridizes under stringent conditions with the DNA sequence of SEQ ID NO:1, or the complement thereof.

3. The isolated DNA of claim 1, wherein said DNA comprises the sequence of SEQ ID NO:1, or the complement thereof.

4. The isolated DNA of claim 1, wherein said DNA is genomic DNA.

5. The isolated DNA of claim 1, wherein said DNA is cDNA.

6. An isolated DNA comprising at least 30 nucleotides in length, comprising (a) a strand which hybridizes under stringent conditions to a DNA having the sequence of the coding region of SEQ ID NO:1, (b) the complement thereof, or (c) a double stranded DNA comprising both (a) and (b).

7. A single or double stranded isolated DNA encoding maspin (SEQ ID NO:2) or the complement of said single-stranded DNA.

8. The isolated DNA of claim 7, wherein said DNA comprises a sequence which hybridises under stringent conditions with the DNA sequence of SEQ ID NO:1, or the complement thereof.

9. A vector comprising the isolated DNA of claim 1.

10. The vector of claim 9, wherein said isolated DNA is genomic DNA.

11. The vector of claim 9, wherein said vector includes an expression control sequence.

12. A vector comprising the isolated DNA of claim 7.

13. The vector of claim 12, wherein the vector includes an expression control sequence linked to said isolated DNA.

14. A cell transfected with the vector of claim 9.

15. The cell of claim 14, wherein said cell expresses said polypeptide.

16. A cell transfected with a vector comprising the isolated DNA of claim 6.

17. A cell transfected with the vector of claim 12.

18. The cell of claim 17, wherein said cell expresses the polypeptide.

19. A purified preparation of maspin (SEQ ID NO:2).

20. An antigenic polypeptide comprising from 10 to 374 amino acid residues of maspin (a protein having the amino acid sequence of SEQ ID NO:2), said polypeptide comprising an epitope of maspin such that an antibody raised against a conjugate of said polypeptide and keyhole limpet hemocyanin forms an immune complex with maspin.

21. An antibody which forms an immune complex with maspin (SEQ ID NO:2).

22. The antibody of claim 21, wherein said antibody is polyclonal.

23. The antibody of claim 21, wherein said antibody is monoclonal.

24. The antibody of claim 21, wherein said antibody is specific for an epitope within the sequence YAKELETVDFKDKLE.

25. The antibody of claim 21, wherein said antibody is specific for an epitope within the sequence GKWMKKFPESETKE.

26. The antibody of claim 21, wherein said antibody is specific for an epitope within the sequence IEVPGARILQHKDEL.

27. A method of making an antibody, comprising the step of immunizing an animal with an antigen comprising an antigenic portion of maspin (SEQ ID NO: 2).

28. An in vitro diagnostic method comprising:
providing an isolated test cell from a given type of epithelial tissue, said isolated test cell being suspected of being cancerous;
contacting the mRNA of said test cell with a single-stranded nucleic acid probe comprising the isolated DNA of claim 6, said DNA being antisense to a segment of SEQ ID NO:1; and
comparing (1) the amount of hybridization of said probe to said mRNA of said test cell, with (2) the amount of hybridization of said probe to the mRNA of a normal control cell from said type of epithelial tissue, wherein an amount of hybridization to the mRNA of said test cell substantially less than the amount obtained with the mRNA of said normal control cell is an indication that said test cell is cancerous.

29. The method of claim 28, wherein said segment is at least 30 nucleotides in length.

30. The method of claim 28, wherein said hybridization is performed *in situ* using a fixed sample of said epithelial tissue.

31. The method of claim 28, wherein said hybridization is performed as a Northern analysis.

32. The method of claim 28, wherein said epithelial tissue is mammary epithelial tissue.

33. An in vitro diagnostic method comprising:
providing an isolated test cell from a given type of epithelial tissue, said isolated test cell being suspected of being cancerous;
contacting proteins of the test cell with the antibody of claim 21; and
comparing (1) the amount of immunocomplex formation by said antibody and said proteins, with (2) the amount of immunocomplex formation by said antibody and the proteins of a normal control cell from said type of epithelial tissue, wherein an amount of immunocomplex formation with the proteins of said test cell substantially less than the amount obtained with the proteins of said normal control cell is an indication that said test cell is cancerous.

34. The method of claim 33, wherein said epithelial tissue is mammary epithelial tissue.

35. The method of claim 33 wherein said antibody is polyclonal.

36. The method of claim 35, wherein said epithelial tissue is mammalian epithelial tissue.

37. The method of claim 33, wherein said antibody is monoclonal.

38. The method of claim 37, wherein said epithelial tissue is mammalian epithelial tissue.

39. The method of claim 33, wherein said antibody is specific for an epitope within an amino acid sequence selected from the group consisting of YAKELETVDFKDKLE, GKWMKKFPESETKE and IEVPGARILQHKDEL.

40. The method of claim 39, wherein said epithelial tissue is mammary epithelial tissue.

41. An in vitro method for screening candidate anticancer compounds, comprising the steps of:
providing a first and a second sample of isolated cells from a carcinoma from a given tissue type, the level of expression of maspin (SEQ ID NO:2) in said carcinoma being substantially less than the level of expression in normal epithelial cells from said type of tissue;
treating said first sample with a candidate compound; and
comparing the level of expression of maspin in said first sample with the level in said second sample, a higher level of expression in said first sample being an indication that said candidate compound is a potential anticancer agent.

42. A polypeptide comprising an amino acid sequence identified in SEQ ID NO:2 or a biologically active fragment thereof for use as a medicament.

43. Use of a polypeptide comprising an amino acid sequence according to SEQ ID NO:2 or a biologically active fragment thereof in the manufacture of a medicament for treating cancer.

44. A nucleic acid encoding a polypeptide comprising an amino acid sequence according to SEQ ID NO:2 and having expression control elements permitting expression of said polypeptide in carcinoma cells for use in treating cancer.

45. Use of a nucleic acid encoding a polypeptide comprising an amino acid sequence according to SEQ ID NO:2 and having expression control elements permitting expression of maspin in carcinoma cells in the manufacture of a medicament for treating cancer.

46. A method for determining whether a test carcinoma cell represents an (a) early-stage or (b) advanced, metastatic carcinoma, said carcinoma being derived from a type of epithelial tissue, in normal cells of which mRNA of maspin (a protein having the sequence of SEQ ID NO:2) is detectably expressed, which method comprises the steps of:
contacting the mRNA of the test carcinoma cell with a single stranded nucleic acid probe comprising the isolated DNA of claim 6, said DNA being antisense to a segment of SEQ ID NO:1; and
determining the amount of hybridization of said probe to said mRNA of said test cell, wherein the absence of consistently detectable hybridization is an indication that said test cell is from an advanced, metastatic tumor.

47. The method of claim 46, wherein an amount of hybridization to the mRNA of said test cell that is consistently detectable but less than that measured in a normal cell of said type of tissue is an indication that said carcinoma is an early stage carcinoma.

48. A method for determining whether a test carcinoma cell represents an (a) early-stage or (b) advanced, metastatic carcinoma, said carcinoma being derived from a type of epithelial tissue, in normal cells of which maspin (a protein having the sequence of SEQ ID NO:2) is detectably expressed, which method comprises the steps of:
contacting the proteins of the test carcinoma cell with the antibody of claim 15; and
determining the amount of immunocomplex formed by said antibody and said proteins, wherein the absence of consistently detectable immunocomplex is an indication that said test cell is from an advanced, metastatic tumor.

49. The method of claim 48, wherein an amount of immunocomplex that is consistently detectable but less than that measured in a normal cell of said type of tissue is an indication that said carcinoma is an early stage carcinoma.

50. The method of claim 48, wherein said antibody is specific for an epitope within an amino acid sequence selected from the group consisting of YAKELETVDFKDKLE, GKWMKKFPESETKE and IEVPGARILQHKDEL.

51. The method of claim 50, wherein an amount of immunocomplex that is consistently detectable but less than that measured in a normal cell of said type of tissue is an indication that said carcinoma is an early stage carcinoma.

52. A method for determining the level of maspin a) protein having the sequence of SEQ ID NO:2) in a biological fluid, comprising the steps of:
obtaining a sample of a biological fluid from an individual;
contacting proteins in said sample with the antibody of claim 21; and
determining the amount of immunocomplex formation by said antibody, said amount being indicative of the level of maspin in said sample.

53. The method of claim 52, wherein said biological fluid is blood, serum, urine, saliva, milk, ductal fluid, tears, or semen.

54. The method of claim 52, wherein said amount is compared to the amount of immunocomplex formation by said antibody in a sample previously or subsequently obtained from said individual.

55. The method of claim 52, wherein said antibody is polyclonal.

56. The method of claim 55, wherein said biological fluid is blood, serum, urine, saliva, milk, ductal fluid, tears, or semen.

57. The method of claim 55, wherein said amount is compared to the amount of immunocomplex formation by said antibody in a sample previously or subsequently obtained from said individual.

58. The method of claim 52, wherein said antibody is monoclonal.

59. The method of claim 58, wherein said biological fluid is blood, serum, urine, saliva, milk, ductal fluid, tears, or semen.

60. The method of claim 58, wherein said amount is compared to the amount of immunocomplex formation by said antibody in a sample previously of subsequently obtained from said individual.

61. The method of claim 52, wherein said antibody is specific for an epitope within an amino acid sequence selected from the group consisting of YAKELETVDFKDKLE, GKWMKKFPESETKE, and IEVPGARILQHKDEL.

62. The method of claim 61, wherein said biological fluid is blood, serum, urine, saliva, milk, ductal fluid, tears, or semen.

63. The method of claim 61, wherein said amount is compared to the amount of immunocomplex formation by said antibody in a sample previously or subsequently obtained from said individual.

64. A hybrid polypeptide comprising (1) maspin (SEQ ID NO:2), or an antigenic fragment thereof, covalently attached to (2) a second polypeptide.

65. An *in vitro* method for identifying a compound which increases maspin (SEQ ID NO:1) expression, comprising:
identifying a first and second cell of a carcinoma as having a maspin expression level less than one third than that of a normal cell from the same type of tissue as the carcinoma cells;
treating the first cell with a test compound; and
determining whether the first cell has a higher level of maspin expression than the second cell, wherein a higher level of expression in the first cell is an indication that the test compound increases maspin expression.

66. A method for screening candidate anticancer compounds, comprising the steps of:
providing a cell in which expression of maspin (SEQ ID NO: 2) is essentially undetectable, but which contains an intact maspin gene;
treating said cell with a candidate anticancer compound; and
determining whether expression of maspin is increased in said cell, wherein an increase in said expression is an indication that said candidate compound is a potential anticancer agent.

## Patentansprüche

1. Isolierte einzel- oder doppelsträngige DNA, die für ein Polypeptid codiert, das mindestens eine 90%ige Sequenzidentität mit Maspin (SEQ ID NO:2) besitzt.

2. Isolierte DNA nach Anspruch 1, worin die DNA eine Sequenz umfasst, die unter stringenten Bedingungen mit der DNA-Sequenz SEQ ID NO:1 oder einer dazu komplementären Sequenz hybridisiert.

3. Isolierte DNA nach Anspruch 1, worin die DNA die Sequenz SEQ ID NO:1 oder die dazu komplementäre Sequenz umfasst.

4. Isolierte DNA nach Anspruch 1, worin die DNA eine genomische DNA ist.

5. Isolierte DNA nach Anspruch 1, worin die DNA eine cDNA ist.

6. Isolierte DNA, umfassend mindestens ein Länge von 30 Nukleotiden, umfassend (a) einen Strang, der unter stringenten Bedingungen mit einer DNA hybridisiert, die die Sequenz der codierenden Region von SEQ ID NO:1 besitzt, (b) die dazu komplementäre Sequenz, oder (c) eine doppelsträngige DNA, die sowohl (a) als auch (b) umfasst.

7. Isolierte einzel- oder doppelsträngige DNA, die für Maspin (SEQ ID NO:2) codiert oder die komplementäre Sequenz der einzelsträngigen DNA.

8. Isolierte DNA nach Anspruch 7, worin die DNA eine Sequenz umfasst, die unter stringenten Bedingungen mit der DNA-Sequenz SEQ ID NO:1 oder mit der dazu komplementären Sequenz hybridisiert.

9. Vektor, umfassend die isolierte DNA nach Anspruch 1.

10. Vektor nach Anspruch 9, worin die isolierte DNA eine genomische DNA ist.

11. Vektor nach Anspruch 9, worin der Vektor eine ExpressionsKontrollsequenz einschließt.

12. Vektor, umfassend die isolierte DNA nach Anspruch 7.

13. Vektor nach Anspruch 12, worin der Vektor eine ExpressionsKontrollsequenz einschließt, die mit der isolierten DNA verknüpft ist.

14. Zelle, transfiziert mit dem Vektor nach Anspruch 9.

15. Zelle nach Anspruch 14, worin die Zelle das Polypeptid exprimiert.

16. Zelle, transfiziert mit einem Vektor, umfassend die isolierte DNA nach Anspruch 6.

17. Zelle, transfiziert mit dem Vektor nach Anspruch 12.

18. Zelle nach Anspruch 17, worin die Zelle das Polypeptid exprimiert.

19. Gereinigte Maspin (SEQ ID NO:2) -Präparation.

20. Antigenes Polypeptid, umfassend von 10 bis 374 Aminosäurereste von Maspin (ein Protein, das die Aminosäuresequenz SEQ ID NO:2 besitzt), wobei das Polypeptid ein Epitop von Maspin umfasst, so dass ein Antikörper, der gegen ein Konjugat aus dem Polypeptid und Hämocyanin der Schlüsselloch-Napfschnecke (Keyhole Limpet) gerichtetet ist, einen Immunkomplex mit Maspin bildet.

21. Antikörper, der mit Maspin (SEQ ID NO:2) einen Immunkomplex bildet.

22. Antikörper nach Anspruch 21, worin der Antikörper polyklonal ist.

23. Antikörper nach Anspruch 21, worin der Antikörper monoklonal ist.

24. Antikörper nach Anspruch 21, worin der Antikörper spezifisch ist für ein Epitop innerhalb der Sequenz YAKELETVDFKDKLE.

25. Antikörper nach Anspruch 21, worin der Antikörper spezifisch ist für ein Epitop innerhalb der Sequenz GKWMKKFPESETKE.

26. Antikörper nach Anspruch 21, worin der Antikörper spezifisch ist für ein Epitop innerhalb der Sequenz IEVPGARILQHKDEL.

27. Verfahren zur Herstellung eines Antikörpers, umfassend den Schritt der Immunisierung eines Tieres mit einem Antigen, das einen antigenen Anteil von Maspin (SEQ ID NO:2) umfasst.

28. Diagostisches *in vitro*-Verfahren, umfassend:
das Bereitstellen einer isolierten Testzelle einer gegebenen Epithelgewebsart, wobei die isolierte Testzelle im Verdacht steht, kanzerös zu sein;
das Kontaktieren der mRNA der Testzelle mit einer einzelsträngigen Nukleinsäuresonde, umfassend die isolierte DNA nach Anspruch 6, worin die DNA bezüglich eines Segments von SEQ ID NO:1 in Antisense-Orientierung vorliegt; und
das Vergleichen (1) des Ausmaßes der Hybridisierung der Sonde mit der mRNA der Testzelle, mit (2) dem Ausmaß der Hybridisierung der Sonde mit der mRNA einer normalen Kontrollzelle der Epithelgewebsart, worin ein Ausmaß der Hybridisierung der mRNA der Testzelle, das wesentlich geringer ist, als das mit der mRNA der normalen Kontrollzelle erhaltene Ausmaß, ein Anzeichen dafür ist, dass die Testzelle kanzerös ist.

29. Verfahren nach Anspruch 28, worin das Segment mindestens eine Länge von 30 Nukleotiden besitzt.

30. Verfahren nach Anspruch 28, worin die Hybridisierung *in situ* unter Verwendung einer fixierten Probe des Epithelgewebes durchgeführt wird.

31. Verfahren nach Anspruch 28, worin die Hybridisierung in Form einer Northern-Analyse durchgeführt wird.

32. Verfahren nach Anspruch 28, worin das Epithelgewebe Brust-Epithelgewebe ist.

33. Diagnostisches *in vitro*-Verfahren, umfassend:
das Bereitstellen einer isolierten Testzelle aus einer gegebenen Epithelgewebsart, wobei die isolierte Testzelle im Verdacht steht, kanzerös zu sein;
das Kontaktieren von Proteinen der Testzelle mit dem Antikörper nach Anspruch 21; und
das Vergleichen (1) des Ausmaßes der Immunkomplex-Bildung des Antikörpers und der Proteine, mit (2) dem Ausmaß einer Immunkomplex-Bildung des Antikörpers und der Proteine einer normalen Kontrollzelle der Epithelgewebsart, worin ein Ausmaß der Immunkomplex-Bildung mit den Proteinen der Testzelle, das wesentlich geringer ist, als das mit den Proteinen der normalen Kontrollzelle erhaltene Ausmaß, ein Anzeichen dafür ist, dass die Testzelle kanzerös ist.

34. Verfahren nach Anspruch 33, worin das Epithelgewebe Brust-Epithelgewebe ist.

35. Verfahren nach Anspruch 33, worin der Antikörper polyklonal ist.

36. Verfahren nach Anspruch 35, worin das Epithelgewebe Brust-Epithelgewebe ist.

37. Verfahren nach Anspruch 33, worin der Antikörper monoklonal ist.

38. Verfahren nach Anspruch 37, worin das Epithelgewebe Brust-Epithelgewebe ist.

39. Verfahren nach Anspruch 33, worin der Antikörper spezifisch ist für ein Epitop innerhalb einer Aminosäuresequenz, ausgewählt aus der Gruppe bestehend aus YAKELETVDFKDKLE, GKWMKKFPESETKE und IEVPGARILQHKDEL.

40. Verfahren nach Anspruch 39, worin das Epithelgewebe Brust-Epithelgewebe ist.

41. *In vitro*-Verfahren zum Screening von möglicherweise gegen Krebs wirkenden Verbindungen , umfassend die Schritte:
des Bereitstellens einer ersten und einer zweiten Probe aus isolierten Zellen von einem Karzinom einer gegebenen Gewebsart, wobei das Expressionsniveau von Maspin (SEQ ID NO:2) in dem Karzinom wesentlich geringer ist als das Expressionsniveau in normalen Epithelzellen der Gewebsart;
des Behandelns der ersten Probe mit einer Testverbindung; und
des Vergleichens des Expressionsniveaus von Maspin in der ersten Probe mit dem Niveau in der zweiten Probe, wobei ein höheres Expressionsniveau in der ersten Probe ein Anzeichen dafür ist, dass die Testverbindung ein potentielles Mittel gegen Krebs ist.

42. Polypeptid, umfassend eine Aminosäuresequenz, die in SEQ ID NO:2 identifiziert ist oder ein biologisch aktives Fragment davon zur Verwendung als ein Medikament.

43. Verwendung eines Polypeptids umfassend eine Aminosäuresequenz nach SEQ ID NO:2 oder ein biologisch aktives Fragment davon bei der Herstellung eines Medikaments zur Behandlung von Krebs.

44. Nukleinsäure, die für ein Polypetid codiert, das eine Aminosäuresequenz gemäß SEQ ID NO:2 umfasst und Expressions-Kontrollelemente besitzt, die eine Expression des Polypeptids in Karzinomzellen zur Behandlung von Krebs ermöglichen.

45. Verwendung einer Nukleinsäure, die für ein Polypetid codiert, das eine Aminosäuresequenz gemäß SEQ ID NO:2 umfasst und Expressions-Kontrollelemente besitzt, die eine Expression von Maspin in Karzinomzellen ermöglichen, bei der Herstellung eines Medikaments zur Behandlung von Krebs.

46. Verfahren zur Bestimmung, ob eine Test-Karzinomzelle (a) ein Karzinom in einem frühen Stadium oder (b) ein fortgeschrittenes metastatisches Karzinom darstellt, wobei das Karzinom von einer Epithelgewebsart abgeleitet ist, in normalen Zellen, worin eine mRNA von Maspin (ein Protein, das die Sequenz SEQ ID NO:2 besitzt) nachweisbar exprimiert wird, wobei das Verfahren die Schritte umfasst:
Kontaktieren der mRNA der Test-Karzinomzelle mit einer einzelsträngigen Nukleinsäuresonde, die die isolierte DNA nach Anspruch 6 umfasst, wobei die DNA eine Antisense-Orientierung zu einem Segment von SEQ ID NO:1 aufweist; und
Bestimmen des Ausmaßes der Hybridisierung der Sonde mit der mRNA der Testzelle, worin die Abwesenheit einer ständig nachweisbaren Hybridisierung ein Anzeichen dafür ist, dass die Testzelle von einem fortgeschrittenen metastatischen Tumor stammt.

47. Verfahren nach Anspruch 46, worin ein Ausmaß der Hybridisierung mit der mRNA der Testzelle, das ständig nachweisbar, aber geringer ist als das in einer normalen Zelle der Gewebsart gemessene Ausmaß, ein Anzeichen dafür ist, dass das Karzinom ein Karzinom in einem frühen Stadium ist.

48. Verfahren zur Bestimmung, ob eine Test-Karzinomzelle (a) ein Karzinom in einem frühen Stadium oder (b) ein fortgeschrittenes metastatisches Karzinom darstellt, wobei das Karzinom von einer Epithelgewebsart abgeleitet ist, in normalen Zellen, in denen Maspin (ein Protein, das die Sequenz SEQ ID NO:2 besitzt) nachweisbar exprimiert wird, wobei das Verfahren die Schritte umfasst:
Kontaktieren des Proteins der Test-Karzinomzelle mit dem Antikörper nach Anspruch 15; und
Bestimmen der Menge des durch den Antikörper und die Proteine gebildeten Immunkomplexes, worin die Abwesenheit eines ständig nachweisbaren Immunkomplexes ein Anzeichen dafür ist, dass die Testzelle von einem fortgeschrittenen metastatischen Tumor stammt.

49. Verfahren nach Anspruch 48, worin eine Menge des Immunkomplexes, die ständig nachweisbar, aber geringer ist als die in einer normalen Zelle der Gewebsart gemessene Menge, ein Anzeichen dafür ist, dass das Karzinom ein Karzinom in einem frühen Stadium ist.

50. Verfahren nach Anspruch 48, worin der Antikörper spezifisch ist für ein Epitop innerhalb einer Aminosäuresequenz, ausgewählt aus der Gruppe bestehend aus YAKELETVDFKDKLE, GKWMKKFPESETKE und IEVPGARILQHKDEL.

51. Verfahren nach Anspruch 50, worin eine Menge des Immunkomplexes, die ständig nachweisbar, aber geringer ist als die in einer normalen Zelle der Gewebsart gemessene Menge, ein Anzeichen dafür ist, dass das Karzinom ein Karzinom in einem frühen Stadium ist.

52. Verfahren zur Bestimmung der Menge an Maspin (ein Protein, das die Sequenz SEQ ID NO:2 besitzt) in einer biologischen Flüssigkeit, umfassend die Schritte:
des Gewinnens einer Probe einer biologischen Flüssigkeit von einem Individuum;
des Kontaktierens von Proteinen in der Probe mit dem Antikörper nach Anspruch 21; und
des Bestimmens des Ausmaßes der Immunkomplex-Bildung durch den Antikörper, wobei von diesem Ausmaß auf die Menge an Maspin in der Probe geschlossen werden kann.

53. Verfahren nach Anspruch 52, worin die biologische Flüssigkeit Blut, Serum, Urin, Speichel, Milch, Ductusflüssigkeit, Tränenflüssigkeit oder Sperma ist.

54. Verfahren nach Anspruch 52, worin das Ausmaß verglichen wird mit dem Ausmaß der Immunkomplex-Bildung durch den Antikörper in einer Probe, die früher oder nachfolgend von dem Individuum gewonnen wurde.

55. Verfahren nach Anspruch 52, worin der Antikörper polyklonal ist.

56. Verfahren nach Anspruch 55, worin die biologische Flüssigkeit Blut, Serum, Urin, Speichel, Milch, Ductusflüssigkeit, Tränenflüssigkeit oder Sperma ist.

57. Verfahren nach Anspruch 55, worin das Ausmaß verglichen wird mit dem Ausmaß der Immunkomplex-Bildung durch den Antikörper in einer Probe, die früher oder nachfolgend von dem Individuum gewonnen wurde.

58. Verfahren nach Anspruch 52, worin der Antikörper monoklonal ist.

59. Verfahren nach Anspruch 58, worin die biologische Flüssigkeit Blut, Serum, Urin, Speichel, Milch, Ductusflüssigkeit, Tränenflüssigkeit oder Sperma ist.

60. Verfahren nach Anspruch 58, worin das Ausmaß verglichen wird mit dem Ausmaß der Immunkomplex-Bildung durch den Antikörper in einer Probe, die früher oder nachfolgend von dem Individuum gewonnen wurde.

61. Verfahren nach Anspruch 52, worin der Antikörper spezifisch ist für ein Epitop innerhalb einer Aminosäuresequenz, ausgewählt aus der Gruppe bestehend aus YAKELETVDFKDKLE, GKWMKKFPESETKE und IEVPGARILQHKDEL.

62. Verfahren nach Anspruch 61, worin die biologische Flüssigkeit Blut, Serum, Urin, Speichel, Milch, Ductusflüssigkeit, Tränenflüssigkeit oder Sperma ist.

63. Verfahren nach Anspruch 61, worin das Ausmaß verglichen wird mit dem Ausmaß der Immunkomplex-Bildung durch den Antikörper in einer Probe, die früher oder nachfolgend von dem Individuum gewonnen wurde.

64. Hybrid-Polypeptid, umfassend (1) Maspin (SEQ ID NO:2) oder ein antigenes Fragment davon, das kovalent mit (2) einem zweiten Polypetid verbunden ist.

65. *In vitro*-Verfahren zur Identifizierung einer Verbindung, die eine Maspin (SEQ ID NO:1) -Expression erhöht, umfassend:
das Identifizieren einer ersten und zweiten Zelle eines Karzinoms, die ein Maspin-Expressionsniveau besitzen, das geringer ist als ein drittel dessen einer normalen Zelle von der gleichen Gewebsart wie die Karzinomzelle;
das Behandeln der ersten Zelle mit einer Testverbindung; und
das Bestimmen, ob die erste Zelle ein höheres Expressionsniveau besitzt als die zweite Zelle, worin ein höheres Expressionsniveau in der ersten Zelle ein Anzeichen dafür ist, dass die Testverbindung die Maspin-Expression erhöht.

66. Verfahren zum Screening von möglicherweise gegen Krebs wirkenden Verbindungen, umfassend die Schritte:
des Bereitstellens einer Zelle, in der eine Maspin- (SEQ ID NO:2) Expression im Wesentlichen nicht nachweisbar ist, die aber ein intaktes Maspin-Gen enthält;
des Behandelns der Zelle mit einer möglicherweise gegen Krebs wirkenden Verbindung; und
des Bestimmens, ob eine Expression von Maspin in der Zelle erhöht ist, wobei ein Anstieg in der Expression ein Anzeichen dafür ist, dass die Testverbindung ein potentielles Mittel gegen Krebs ist.

## Revendications

1. ADN mono- ou bicaténaire isolé codant pour un polypeptide ayant une identité de séquence d'au moins 90 % avec la maspine (SEQ ID N° 2).

2. ADN isolé suivant la revendication 1, ledit ADN comprenant une séquence qui s'hybride, dans des conditions drastiques, avec la séquence d'ADN de la SEQ ID N° 1, ou son complément.

3. ADN isolé suivant la revendication 1, ledit ADN comprenant la séquence de la SEQ ID N° 1, ou son complément.

4. ADN isolé suivant la revendication 1, ledit ADN consistant en ADN génomique.

5. ADN isolé suivant la revendication 1, ledit ADN consistant en ADNc.

6. ADN isolé ayant une longueur d'au moins 30 nucléotides, comprenant (a) un brin qui s'hybride, dans des conditions drastiques, à un ADN ayant la séquence de la région codante de la SEQ ID N° 1, (b) son complément, ou (c) un ADN bicaténaire comprenant à la fois (a) et (b).

7. ADN mono- ou bicaténaire isolé codant pour la maspine (SEQ ID N° 2) ou le complément dudit ADN monocaténaire.

8. ADN isolé suivant la revendication 7, ledit ADN comprenant une séquence qui s'hybride, dans des conditions drastiques, avec la séquence d'ADN de la SEQ ID N^{c} 1, ou son complément.

9. Vecteur comprenant l'ADN isolé suivant la revendication 1.

10. Vecteur suivant la revendication 9, dans lequel l'ADN isolé consiste en ADN génomique.

11. Vecteur suivant la revendicaiton 9, ledit vecteur comprenant une séquence de régulation d'expression.

12. Vecteur comprenant l'ADN isolé suivant la revendication 7.

13. Vecteur suivant la revendication 12, ledit vecteur comprenant une séquence de régulation d'expression liée audit ADN isolé.

14. Cellule transfectée avec le vecteur suivant la revendication 9.

15. Cellule suivant la revendication 14, ladite cellule exprimant ledit polypeptide.

16. Cellule transfectée avec un vecteur comprenant l'ADN isolé suivant la revendication 6.

17. Cellule transfectée avec le vecteur suivant la revendication 12.

18. Cellule suivant la revendication 17, ladite cellule exprimant le polypeptide.

19. Préparation purifiée de maspine (SEQ ID N° 2).

20. Polypeptide antigénique comprenant 10 à 374 résidus d'amino-acides de la maspine (une protéine ayant la séquence d'amino-acides de la SEQ ID N° 2), ledit polypeptide comprenant un épitope de maspine de telle sorte qu'un anticorps engendré contre un conjugué dudit polypeptide et d'hémocyanine de patelle du genre Fissurella forme un complexe immun avec la maspine.

21. Anticorps qui forme un complexe immun avec la maspine (SEQ ID N° 2).

22. Anticorps suivant la revendication 21, ledit anticorps étant polyclonal.

23. Anticorps suivant la revendication 21, ledit anticorps étant monoclonal.

24. Anticorps suivant la revendication 21, ledit anticorps étant spécifique d'un épitope dans la séquence YAKELETVDFKDKLE.

25. Anticorps suivant la revendication 21, ledit anticorps étant spécifique d'un épitope dans la séquence GKWMKKFPESETKE.

26. Anticorps suivant la revendication 21, ledit anticorps étant spécifique d'un épitope dans la séquence IEVPGARILQHKDEL.

27. Procédé pour la préparation d'un anticorps, comprenant l'étape consistant à immuniser un animal avec un antigène comprenant une partie antigénique de la maspine (SEQ ID N° 2).

28. Méthode de diagnostic in vitro, comprenant les étapes consistant :
à prendre une cellule d'essai isolée à partir d'un type donné de tissu épithélial, ladite cellule d'essai isolée étant soupçonnée être une cellule cancéreuse ;
à mettre en contact l'ARNm de ladite cellule d'essai avec une sonde d'acide nucléique monocaténaire comprenant l'ADN isolé suivant la revendication 6, ledit ADN étant antisens par rapport à un segment de la SEQ ID N° 1 ; et
à comparer (1) le degré d'hybridation de ladite sonde audit ARNm de ladite cellule d'essai, avec (2) le degré d'hybridation de ladite sonde à l'ARNm d'une cellule témoin normale provenant dudit type de tissu épithélial, un degré d'hybridation à l'ARNm de ladite cellule d'essai substantiellement inférieur au degré obtenu avec l'ARNm de ladite cellule témoin normale étant une indication que ladite cellule d'essai est cancéreuse.

29. Méthode suivant la revendication 28, dans laquelle le segment a une longueur d'au moins 30 nucléotides.

30. Méthode suivant la revendication 28, dans laquelle l'hybridation est effectuée in situ en utilisant un échantillon fixé dudit tissu épithélial.

31. Méthode suivant la revendication 28, dans laquelle l'hybridation est effectuée sous forme d'une analyse de Northern.

32. Méthode suivant la revendication 28, dans laquelle le tissu épithélial consiste en un tissu épithélial de mammifère.

33. Méthode de diagnostic in vitro, comprenant les étapes consistant :
à prendre une cellule d'essai isolée à partir d'un type donné de tissu épithélial, ladite cellule d'essai isolée étant soupçonnée être une cellule cancéreuse ;
à mettre en contact les protéines de la cellule d'essai avec l'anticorps suivant la revendication 21 ; et
à comparer (1) la quantité de complexe immun formée par ledit anticorps et lesdites protéines avec (2) la quantité de complexe immun formée par ledit anticorps et les protéines d'une cellule témoin normale provenant dudit type de tissu épithélial, une quantité de complexe immun formée avec les protéines de ladite cellule d'essai sensiblement inférieure à la quantité obtenue avec les protéines de ladite cellule témoin normale étant une indication que ladite cellule d'essai est cancéreuse.

34. Méthode suivant la revendication 33, dans laquelle le tissu épithélial est un tissu épithélial de mammifère.

35. Méthode suivant la revendication 33, dans laquelle l'anticorps est polyclonal.

36. Méthode suivant la revendication 35, dans laquelle le tissu épithélial est un tissu épithélial de mammifère.

37. Méthode suivant la revendication 33, dans laquelle l'anticorps est monoclonal.

38. Méthode suivant la revendication 37, dans laquelle le tissu épithélial est un tissu épithélial de mammifère.

39. Méthode suivant la revendication 33, dans laquelle l'anticorps est spécifique d'un épitope dans une séquence d'amino-acides choisie dans le groupe consistant en YAKELETVDFKDKLE, GKWMKKFPESETKE et IEVPGARILQHKDEL.

40. Méthode suivant la revendication 39, dans laquelle le tissu épithélial est un tissu épithélial de mammifère.

41. Méthode in vitro pour dépister des composés anticancéreux candidats, comprenant les étapes consistant :
à prendre des premier et second échantillons de cellules isolées provenant d'un carcinome d'un type de tissu donné, le degré d'expression de maspine (SEQ ID N° 2) dans ledit carcinome étant sensiblement inférieur au degré d'expression dans les cellules épithéliales normales provenant dudit type de tissu ;
à traiter ledit premier échantillon avec un composé candidat ; et
à comparer le degré d'expression de maspine dans ledit premier échantillon avec le degré dans ledit second échantillon, un plus haut degré d'expression dans ledit premier échantillon étant une indication que ledit composé candidat est un agent anticancéreux potentiel.

42. Polypeptide comprenant une séquence d'amino-acides identifiée dans la SEQ ID N° 2 ou un de ses fragments biologiquement actifs destiné à être utilisé comme médicament.

43. Utilisation d'un polypeptide comprenant une séquence d'amino-acides suivant la SEQ ID N° 2 ou d'un de ses fragments biologiquement actifs dans la production d'un médicament destiné au traitement du cancer.

44. Acide nucléique codant pour un polypeptide comprenant une séquence d'amino-acides suivant la SEQ ID N° 2 et comprenant des éléments de régulation d'expression permettant l'expression dudit polypeptide dans des cellules de carcinome à des fins d'utilisation dans le traitement du cancer.

45. Utilisation d'un acide nucléique codant pour un polypeptide comprenant une séquence d'amino-acides suivant la SEQ ID N° 2 et comprenant des éléments de régulation d'expression permettant l'expression de maspine dans des cellules de carcinome dans la production d'un médicament destiné au traitement du cancer.

46. Méthode pour déterminer si une cellule de carcinome d'essai représente (a) un stade précoce ou (b) un carcinome métastatique avancé, ledit carcinome étant dérivé d'un type de tissu épithélial, dans les cellules normales duquel l'ARNm de maspine (une protéine ayant la séquence de la SEQ ID N° 2) est exprimé de manière détectable, méthode qui comprend les étapes consistant :
à mettre en contact l'ARNm de la cellule de carcinome d'essai avec une sonde d'acide nucléique monocaténaire comprenant l'ADN isolé suivant la revendication 6, ledit ADN étant antisens par rapport à un segment de la SEQ ID N° 1 ; et
à déterminer le degré d'hybridation de ladite sonde audit ARNm de ladite cellule d'essai, l'absence d'hybridation détectable de manière uniforme étant une indication que ladite cellule d'essai provient d'une tumeur métastatique avancée.

47. Méthode suivant la revendication 46, dans laquelle un degré d'hybridation à l'ARNm de la cellule d'essai qui est uniformément détectable mais inférieur à celui mesuré dans une cellule normale dudit type de tissu est une indication que ledit carcinome est un carcinome à un stade précoce.

48. Méthode pour déterminer si une cellule de carcinome d'essai représente (a) un stade précoce ou (b) un carcinome métastatique avancé, ledit carcinome étant dérivé d'un type de tissu épithélial, dans les cellules normales duquel la maspine (une protéine ayant la séquence de la SEQ ID N° 2) est exprimée de manière détectable, méthode qui comprend les étapes consistant :
à mettre en contact les protéines de la cellule de carcinome d'essai avec l'anticorps suivant la revendication 15 ; et
à déterminer la quantité de complexe immun formée par ledit anticorps et lesdites protéines, l'absence de complexe immun détectable de manière constante étant une indication que ladite cellule d'essai provient d'une tumeur métastatique avancée.

49. Méthode suivant la revendication 48, dans laquelle une quantité de complexe immun qui est détectable de manière uniforme mais inférieure à celle mesurée dans une cellule normale dudit type de tissu est une indication que ledit carcinome est un carcinome à un stade précoce.

50. Méthode suivant la revendication 48, dans laquelle l'anticorps est spécifique d'un épitope dans une séquence d'amino-acides choisie dans le groupe consistant en YAKELETVDFKDKLE, GKWMKKFPESETKE et IEVPGARILQHKDEL.

51. Méthode suivant la revendication 50, dans laquelle une quantité de complexe immun qui est détectable de manière uniforme mais inférieure à celle mesurée dans une cellule normale dudit type de tissu est une indication que ledit carcinome est un carcinome à un stade précoce.

52. Méthode pour la détermination de la quantité de maspine (une protéine ayant la séquence de la SEQ ID N° 2) dans un fluide biologique, comprenant les étapes consistant :
à prélever un échantillon d'un fluide biologique chez un individu ;
à mettre en contact les protéines présentes dans ledit échantillon avec l'anticorps suivant la revendication 21 ; et
à déterminer la quantité de complexe immun formée par ledit anticorps, ladite quantité étant une indication de la quantité de maspine dans ledit échantillon.

53. Méthode suivant la revendication 52, dans laquelle le fluide biologique est le sang, le sérum, l'urine, la salive, le lait, un fluide ductal, les larmes ou le sperme.

54. Méthode suivant la revendication 52, dans laquelle la quantité est comparée à la quantité de complexe immun formée par ledit anticorps dans un échantillon obtenu antérieurement ou ultérieurement à partir dudit individu.

55. Méthode suivant la revendication 52, dans laquelle l'anticorps est polyclonal.

56. Méthode suivant la revendication 55, dans laquelle le fluide biologique est le sang, le sérum, l'urine, la salive, le lait, un fluide ductal, les larmes ou le sperme.

57. Méthode suivant la revendication 55, dans laquelle ladite quantité est comparée à la quantité de complexe immun formée par ledit anticorps dans un échantillon obtenu antérieurement ou ultérieurement à partir dudit individu.

58. Méthode suivant la revendication 52, dans laquelle l'anticorps est monoclonal.

59. Méthode suivant la revendication 58, dans laquelle le fluide biologique est le sang, le sérum, l'urine, la salive, le lait, un fluide ductal, les larmes ou le sperme.

60. Méthode suivant la revendication 58, dans laquelle ladite quantité est comparée à la quantité de complexe immun formée par ledit anticorps dans un échantillon obtenu antérieurement ou ultérieurement partir dudit individu.

61. Méthode suivant la revendication 52, dans laquelle l'anticorps est spécifique d'un épitope dans une séquence d'amino-acides choisie dans le groupe consistant en YAKELETVDFKDKLE, GKWMKKFPESETKE et IEVPGARILQHKDEL.

62. Méthode suivant la revendication 61, dans laquelle le fluide biologique est le sang, le sérum, l'urine, la salive, le lait, un fluide ductal, les larmes ou le sperme.

63. Méthode suivant la revendication 61, dans laquelle ladite quantité est comparée à la quantité de complexe immun formée par ledit anticorps dans un échantillon obtenu antérieurement ou ultérieurement à partir dudit individu.

64. Polypeptide hybride comprenant (1) la maspine (SEQ ID N° 2), ou un de ses fragments antigéniques, fixé par covalence à (2) à un second polypeptide.

65. Méthode in vitro pour l'identification d'un composé qui accroît l'expression de la maspine (SEQ ID N° 1), comprenant les étapes consistant :
à identifier des première et seconde cellules d'un carcinome en tant que cellule ayant un degré d'expression de maspine inférieur à un tiers de celui d'une cellule normale provenant du même type de tissu que les cellules de carcinome ;
à traiter la première cellule avec un composé d'essai ; et
à déterminer si la première cellule a un degré d'expression de maspine supérieur à celui de la seconde cellule, un plus haut degré d'expression dans la première cellule étant une indication que le composé d'essai augmente l'expression de la maspine.

66. Méthode pour sélectionner des composés anticancéreux candidats, comprenant les étapes consistant :
à prendre une cellule dans laquelle l'expression de la maspine (SEQ ID N° 2) est pratiquement indétectable, mais qui contient un gène de maspine intact ;
à traiter ladite cellule avec un composé anticancéreux candidat ; et
à déterminer si l'expression de la maspine est accrue dans ladite cellule, une augmentation de ladite expression étant une indication que le composé candidat est un agent anticancéreux potentiel.


### SEQUENCE LISTING

(1) GENERAL INFORMATION:
   (i) APPLICANT: Sager, Ruth
   (ii) TITLE OF INVENTION: MASPIN, A NOVEL SERPIN WITH TUMOR SUPPRESSING ACTIVITY
   (iii) NUMBER OF SEQUENCES: 8
   (iv) CORRESPONDENCE ADDRESS:
      (A) ADDRESSEE: Fish & Richardson
      (B) STREET: 225 Franklin Street
      (C) CITY: Boston
      (D) STATE: Massachusetts
      (E) COUNTRY: U.S.A.
      (F) ZIP: 02110-2804
   (v) COMPUTER READABLE FORM:
      (A) MEDIUM TYPE: 3.5" Diskette, 1.44 Mb
      (B) COMPUTER: IBM PS/2 Model 50Z or 55SX
      (C) OPERATING SYSTEM: MS-DOS (Version 5.0)
      (D) SOFTWARE: WordPerfect (Version 5.1)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 07/938,823
      (B) FILING DATE: 09/01/92

      (A) APPLICATION NUMBER: 07/844,296
      (B) FILING DATE: 02/28/92

      (A) APPLICATION NUMBER: 07/662,216
      (B) FILING DATE: 02/28/91
   (viii) ATTORNEY/AGENT INFORMATION:
      (A) NAME: Fraser, Janis K.
      (B) REGISTRATION NUMBER: 34,819
      (C) REFERENCE/DOCKET NUMBER: 00530/072001
   (ix) TELECOMMUNICATION INFORMATION:
      (A) TELEPHONE: (617) 542-5070
      (B) TELEFAX: (617) 542-8906
      (C) TELEX: 200154
(2) INFORMATION FOR SEQUENCE IDENTIFICATION NUMBER: 1:
   (i) SEQUENCE CHARACTERISTICS:
      (A) LENGTH: 2584
      (B) TYPE: nucleic acid
      (C) STRANDEDNESS: double
      (D) TOPOLOGY: linear
   (xi) SEQUENCE DESCRIPTION: SEQ ID NO: 1: 
GGCACGAGTT GTGCTCCTCG CTTGCCTGTT CCTTTTCCAC GCATTTTCCA GGATAACTGT 60

GACTCCAGGC CCGCA ATG GAT GCC CTG CAA CTA GCA AAT TCG GCT TTT GCC 111
Met Asp Ala Leu Gln Leu Ala Asn Ser Ala Phe Ala
1               5                   10

GTT GAT CTG TTC AAA CAA CTA TGT GAA AAG GAG CCA CTG GGC AAT GTC 159
Val Asp Leu Phe Lys Gln Leu Cys Glu Lys Glu Pro Leu Gly Asn Val
        15                  20                  25

CTC TTC TCT CCA ATC TGT CTC TCC ACC TCT CTG TCA CTT GCT CAA GTG 207
Leu Phe Ser Pro Ile Cys Leu Ser Thr Ser Leu Ser Leu Ala Gln Val
    30                  35                  40

GGT GCT AAA GGT GAC ACT GCA AAT GAA ATT GGA CAG GTT CTT CAT TTT 255
Gly Ala Lys Gly Asp Thr Ala Asn Glu Ile Gly Gln Val Leu His Phe
45                  50                  55                  60

GAA AAT GTC AAA GAT ATA CCC TTT GGA TTT CAA ACA GTA ACA TCG GAT 303
Glu Asn Val Lys Asp Ile Pro Phe Gly Phe Gln Thr Val Thr Ser Asp
                65                  70                  75

GTA AAC AAA CTT AGT TCC TTT TAC TCA CTG AAA CTA ATC AAG CGG CTC 351
Val Asn Lys Leu Ser Ser Phe Tyr Ser Leu Lys Leu Ile Lys Arg Leu
            80                  85                  90

TAC GTA GAC AAA TCT CTG AAT CTT TCT ACA GAG TTC ATC AGC TCT ACG 399
Tyr Val Asp Lys Ser Leu Asn Leu Ser Thr Glu Phe Ile Ser Ser Thr
        95                  100                 105

AAG AGA CCC TAT GCA AAG GAA TTG GAA ACT GTT GAC TTC AAA GAT AAA 447
Lys Arg Pro Tyr Ala Lys Glu Leu Glu Thr Val Asp Phe Lys Asp Lys
    110                 115                 120

TTG GAA GAA ACG AAA GGT CAG ATC AAC AAC TCA ATT AAG GAT CTC ACA 495
Leu Glu Glu Thr Lys Gly Gln Ile Asn Asn Ser Ile Lys Asp Leu Thr
125                 130                 135                 140

GAT GGC CAC TTT GAG AAC ATT TTA GCT GAC AAC AGT GTG AAC GAC CAG 543
Asp Gly His Phe Glu Asn Ile Leu Ala Asp Asn Ser Val Asn Asp Gln
                145                 150                 155

ACC AAA ATC CTT GTG GTT AAT GCT GCC TAC TTT GTT GGC AAG TGG ATG 591
Thr Lys Ile Leu Val Val Asn Ala Ala Tyr Phe Val Gly Lys Trp Met
            160                 165                 170

AAG AAA TTT CCT GAA TCA GAA ACA AAA GAA TGT CCT TTC AGA CTC AAC 639
Lys Lys Phe Pro Glu Ser Glu Thr Lys Glu Cys Pro Phe Arg Leu Asn
        175                 180                 185

AAG ACA GAC ACC AAA CCA GTG CAG ATG ATG AAC ATG GAG GCC ACG TTC 687
Lys Thr Asp Thr Lys Pro Val Gln Met Met Asn Met Glu Ala Thr Phe
    190                 195                 200

TGT ATG GGA AAC ATT GAC AGT ATC AAT TGT AAG ATC ATA GAG CTT CCT 735
Cys Met Gly Asn Ile Asp Ser Ile Asn Cys Lys Ile Ile Glu Leu Pro
205                 210                 215                 220
 
TTT CAA AAT AAG CAT CTC AGC ATG TTC ATC CTA CTA CCC AAG GAT GTG 783
Phe Gln Asn Lys His Leu Ser Met Phe Ile Leu Leu Pro Lys Asp Val
                225                 230                 235

GAG GAT GAG TCC ACA GGC TTG GAG AAG ATT GAA AAA CAA CTC AAC TCA 831
Glu Asp Glu Ser Thr Gly Leu Glu Lys Ile Glu Lys Gln Leu Asn Ser
            240                 245                 250

GAG TCA CTG TCA CAG TGG ACT AAT CCC AGC ACC ATG GCC AAT GCC AAG 879
Glu Ser Leu Ser Gln Trp Thr Asn Pro Ser Thr Met Ala Asn Ala Lys
        255                 260                 265

GTC AAA CTC TCC ATT CCA AAA TTT AAG GTG GAA AAG ATG ATT GAT CCC 927
Val Lys Leu Ser Ile Pro Lys Phe Lys Val Glu Lys Met Ile Asp Pro
    270                 275                 280

AAG GCT TGT CTG GAA AAT CTA GGG CTG AAA CAT ATC TTC AGT GAA GAC 975
Lys Ala Cys Leu Glu Asn Leu Gly Leu Lys His Ile Phe Ser Glu Asp
285                 290                 295                 300

ACA TCT GAT TTC TCT GGA ATG TCA GAG ACC AAG GGA GTG GCC CTA TCA 1023
Thr Ser Asp Phe Ser Gly Met Ser Glu Thr Lys Gly Val Ala Leu Ser
                305                 310                 315

AAT GTT ATC CAC AAA GTG TGC TTA GAA ATA ACT GAA GAT GGT GGG GAT 1071
Asn Val Ile His Lys Val Cys Leu Glu Ile Thr Glu Asp Gly Gly Asp
            320                 325                 330

TCC ATA GAG GTG CCA GGA GCA CGG ATC CTG CAG CAC AAG GAT GAA TTG 1119
Ser Ile Glu Val Pro Gly Ala Arg Ile Leu Gln His Lys Asp Glu Leu
        335                 340                 345

AAT GCT GAC CAT CCC TTT ATT TAC ATC ATC AGG CAC AAC AAA ACT CGA 1167
Asn Ala Asp His Pro Phe Ile Tyr Ile Ile Arg His Asn Lys Thr Arg
    350                 355                 360

AAC ATC ATT TTC TTT GGC AAA TTC TGT TCT CCT TAAGTGGCAT AGCCCATGTT 1220
Asn Ile Ile Phe Phe Gly Lys Phe Cys Ser Pro
365                 370                 375

AAGTCCTCCC TGACTTTTCT GTGGATGCCG ATTTCTGTAA ACTCTGCATC CAGAGATTCA 1280

TTTTCTAGAT ACAATAAATT GCTAATGTTG CTGGATCAGG AAGCCGCCAG TACTTGTCAT 1340

ATGTAGCCTT CACACAGATA GACCTTTTTT TTTTTCCAAT TCTATCTTTT GTTTCCTTTT 1400

TTCCCATAAG ACAATGACAT ACGCTTTTAA TGAAAAGGAA TCACGTTAGA GGAAAAATAT 1460

TTATTCATTA TTTGTCAAAT TGTCCGGGGT AGTTGGCAGA AATACAGTCT TCCACAAAGA 1520

AAATTCCTAT AAGGAAGATT TGGAAGCTCT TCTTCCCAGC ACTATGCTTT CCTTCTTTGG 1580

GATAGAGAAT GTTCCAGACA TTCTCGCTTC CCTGAAAGAC TGAAGAAAGT GTAGTGCATG 1640

GGACCCACGA AACTGCCCTG GCTCCAGTGA AACTTGGGCA CATGCTCAGG CTACTATAGG 1700

TCCAGAAGTC CTTATGTTAA GCCCTGGCAG GCAGGTGTTT ATTAAAATTC TGAATTTTGG 1760

GGATTTTCAA AAGATAATAT TTTACATACA CTGTATGTTA TAGAACTTCA TGGATCAGAT 1820

CTGGGGCAGC AACCTATAAA TCAACACCTT AATATGCTGC AACAAAATGT AGAATATTCA 1880
 
GACAAAATGG ATACATAAAG ACTAAGTAGC CCATAAGGGG TCAAAATTTG CTGCCAAATG 1940

CGTATGCCAC CAACTTACAA AAACACTTCG TTCGCAGAGC TTTTCAGATT GTGGAATGTT 2000

GGATAAGGAA TTATAGACCT CTAGTAGCTG AAATGCAAGA CCCCAAGAGG AAGTTCAGAT 2060

CTTAATATAA ATTCACTTTC ATTTTTGATA GCTGTCCCAT CTGGTCATGT GGTTGGCACT 2120

AGACTGGTGG CAGGGGCTTC TAGCTGACTC GCACAGGGAT TCTCACAATA GCCGATATCA 2180

GAATTTGTGT TGAAGGAACT TGTCTCTTCA TCTAATATGA TAGCGGGAAA AGGAGAGGAA 2240

ACTACTGCCT TTAGAAAATA TAAGTAAAGT GATTAAAGTG CTCACGTTAC CTTGACACAT 2300

AGTTTTTCAG TCTATGGGTT TAGTTACTTT AGATGGCAAG CATGTAACTT ATATTAATAG 2360

TAATTTGTAA AGTTGGGTGG ATAAGCTATC CCTGTTGCCG GTTCATGGAT TACTTCTCTA 2420

TAAAAAATAT ATATTTACCA AAAAATTTTG TGACATTCCT TCTCCCATCT CTTCCTTGAC 2480

ATGCATTGTA AATAGGTTCT TCTTGTTCTG AGATTCAATA TTGAATTTCT CCTATGCTAT 2540

TGACAATAAA ATATTATTGA ACTACCAAAA AAAAAAAAAA AAAA 2584
(2) INFORMATION FOR SEQUENCE IDENTIFICATION NUMBER: 2:
   (i) SEQUENCE CHARACTERISTICS:
      (A) LENGTH: 375
      (B) TYPE: amino acid
      (C) STRANDEDNESS:
      (D) TOPOLOGY: linear
   (xi) SEQUENCE DESCRIPTION: SEQ ID NO: 2: 
Met Asp Ala Leu Gln Leu Ala Asn Ser Ala Phe Ala Val Asp Leu Phe
1               5                   10                  15

Lys Gln Leu Cys Glu Lys Glu Pro Leu Gly Asn Val Leu Phe Ser Pro
            20                  25                  30

Ile Cys Leu Ser Thr Ser Leu Ser Leu Ala Gln Val Gly Ala Lys Gly
        35                  40                  45

Asp Thr Ala Asn Glu Ile Gly Gln Val Leu His Phe Glu Asn Val Lys
    50                  55                  60

Asp Ile Pro Phe Gly Phe Gln Thr Val Thr Ser Asp Val Asn Lys Leu
65                  70                  75                  80

Ser Ser Phe Tyr Ser Leu Lys Leu Ile Lys Arg Leu Tyr Val Asp Lys
                85                  90                  95

Ser Leu Asn Leu Ser [?]hr Glu Phe Ile Ser Ser Thr Lys Arg Pro Tyr
            100                 105                 110

Ala Lys Glu Leu Glu Thr Val Asp Phe Lys Asp Lys Leu Glu Glu Thr
        115                 120                 125

Lys Gly Gln Ile Asn Asn Ser Ile Lys Asp Leu Thr Asp Gly His Phe
    130                 135                 140
 
Glu Asn Ile Leu Ala Asp Asn Ser Val Asn Asp Gln Thr Lys Ile Leu
145                 150                 155                 160

Val Val Asn Ala Ala Tyr Phe Val Gly Lys Trp Met Lys Lys Phe Pro
                165                 170                 175

Glu Ser Glu Thr Lys Glu Cys Pro Phe Arg Leu Asn Lys Thr Asp Thr
            180                 185                 190

Lys Pro Val Gln Met Met Asn Met Glu Ala Thr Phe Cys Met Gly Asn
        195                 200                 205

Ile Asp Ser Ile Asn Cys Lys Ile Ile Glu Leu Pro Phe Gln Asn Lys
    210                 215                 220

His Leu Ser Met Phe Ile Leu Leu Pro Lys Asp Val Glu Asp Glu Ser
225                 230                 235                 240

Thr Gly Leu Glu Lys Ile Glu Lys Gln Leu Asn Ser Glu Ser Leu Ser
                245                 250                 255

Gln Trp Thr Asn Pro Ser Thr Met Ala Asn Ala Lys Val Lys Leu Ser
            260                 265                 270

Ile Pro Lys Phe Lys Val Glu Lys Met Ile Asp Pro Lys Ala Cys Leu
        275                 280                 285

Glu Asn Leu Gly Leu Lys His Ile Phe Ser Glu Asp Thr Ser Asp Phe
    290                 295                 300

Ser Gly Met Ser Glu Thr Lys Gly Val Ala Leu Ser Asn Val Ile His
305                 310                 315                 320

Lys Val Cys Leu Glu Ile Thr Glu Asp Gly Gly Asp Ser Ile Glu Val
                325                 330                 335

Pro Gly Ala Arg Ile Leu Gln His Lys Asp Glu Leu Asn Ala Asp His
            340                 345                 350

Pro Phe Ile Tyr Ile Ile Arg His Asn Lys Thr Arg Asn Ile Ile Phe
        355                 360                 365

Phe Gly Lys Phe Cys Ser Pro
    370                 375
(2) INFORMATION FOR SEQUENCE IDENTIFICATION NUMBER: 3:
   (i) SEQUENCE CHARACTERISTICS:
      (A) LENGTH: 418
      (B) TYPE: amino acid
      (C) STRANDEDNESS:
      (D) TOPOLOGY: linear
   (xi) SEQUENCE DESCRIPTION: SEQ ID NO: 3: 

1               5                   10                  15


            20                  25                  30
 

        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310                 315                 320


                325                 330                 335


            340                 345                 350


        355                 360                 365
 

    370                 375                 380


385                 390                 395                 400


                405                 410                 415


(2) INFORMATION FOR SEQUENCE IDENTIFICATION NUMBER: 4:
   (i) SEQUENCE CHARACTERISTICS:
      (A) LENGTH: 379
      (B) TYPE: amino acid
      (C) STRANDEDNESS:
      (D) TOPOLOGY: linear
   (xi) SEQUENCE DESCRIPTION: SEQ ID NO: 4: 
Met Glu Gln Leu Ser Ser Ala Asn Thr Arg Phe Ala Leu Asp Leu Phe
1               5                   10                  15

Leu Ala Leu Ser Glu Asn Asn Pro Ala Gly Asn Ile Phe Ile Ser Pro
            20                  25                  30

Phe Ser Ile Ser Ser Ala Met Ala Met Val Phe Leu Gly Thr Arg Gly
        35                  40                  45

Asn Thr Ala Ala Gln Leu Ser Lys Thr Phe His Phe Asn Thr Val Glu
    50                  55                  60

Glu Val His Ser Arg Phe Gln Ser Leu Asn Ala Asp Ile Asn Lys Arg
65                  70                  75                  80

Gly Ala Ser Tyr Ile Leu Lys Leu Ala Asn Arg Leu Tyr Gly Glu Lys
                85                  90                  95

Thr Tyr Asn Phe Leu Pro Glu Phe Leu Val Ser Thr Gln Lys Thr Tyr
            100                 105                 110

Gly Ala Asp Leu Ala Ser Val Asp Phe Gln His Ala Ser Glu Asp Ala
        115                 120                 125

Arg Lys Thr Ile Asn Gln Trp Val Lys Gly Gln Thr Glu Gly Lys Ile
    130                 135                 140

Pro Glu Leu Leu Ala Ser Gly Met Val Asp Asn Met Thr Lys Leu Val
145                 150                 155                 160

Leu Val Asn Ala Ile Tyr Phe Lys Gly Asn Trp Lys Asp Lys Phe Met
                165                 170                 175

Lys Glu Ala Thr Thr Asn Ala Pro Phe Arg Leu Asn Lys Lys Asp Arg
            180                 185                 190

Lys Thr Val Lys Met Met Tyr Gln Lys Lys Lys Phe Ala Tyr Gly Tyr
        195                 200                 205
 
Ile Glu Asp Leu Lys Cys Arg Val Leu Glu Leu Pro Tyr Gln Gly Glu
    210                 215                 220

Glu Leu Ser Met Val Ile Leu Leu Pro Asp Asp Ile Glu Asp Glu Ser
225                 230                 235                 240

Thr Gly Leu Lys Lys Ile Glu Glu Gln Leu Thr Leu Glu Lys Leu His
                245                 250                 255

Glu Trp Thr Lys Pro Glu Asn Leu Asp Phe Ile Glu Val Asn Val Ser
            260                 265                 270

Leu Pro Arg Phe Lys Leu Glu Glu Ser Tyr Thr Leu Asn Ser Asp Leu
        275                 280                 285

Ala Arg Leu Gly Val Gln Asp Leu Phe Asn Ser Ser Lys Ala Asp Leu
    290                 295                 300

Ser Gly Met Ser Gly Ala Arg Asp Ile Phe Ile Ser Lys Ile Val His
305                 310                 315                 320

Lys Ser Phe Val Glu Val Asn Glu Glu Gly Thr Glu Ala Ala Ala Ala
                325                 330                 335

Thr Ala Gly Ile Ala Thr Phe Cys Met Leu Met Pro Glu Glu Asn Phe
            340                 345                 350

Thr Ala Asp His Pro Phe Leu Phe Phe Ile Arg His Asn Ser Ser Gly
        355                 360                 365

Ser Ile Leu Phe Leu Gly Arg Phe Ser Ser Pro
    370                 375
(2) INFORMATION FOR SEQUENCE IDENTIFICATION NUMBER : 5:
   (i) SEQUENCE CHARACTERISTICS:
      (A) LENGTH: 375
      (B) TYPE: amino acid
      (C) STRANDELNESS:
      (D) TOPOLOGY: linear
   (xi) SEQUENCE DESCRIPTION: SEQ ID NO: 5: 
Gly Ser Ile Gly Ala Ala Ser Met Glu Phe Cys Phe Asp Val Phe Lys
1               5                   10                  15

Glu Leu Lys Val His His Ala Asn Glu Asn Ile Phe Tyr Cys Pro Ile
            20                  25                  30

Ala Ile Met Ser Ala Leu Ala Met Val Tyr Leu Gly Ala Lys Asp Ser
        35                  40                  45

Thr Arg Thr Gln Ile Asn Lys Val Val Arg Phe Asp Lys Leu Pro Gly
    50                  55                  60

Phe Gly Asp Ser Ile Glu Ala Gln Cys Gly Thr Ser Val Asn Val His
65                  70                  75                  80

Ser Ser Leu Arg Asp Ile Leu Asn Gln Ile Thr Lys Pro Asn Asp Val
                85                  90                  95
 
Tyr Ser Phe Ser Leu Ala Ser Arg Leu Tyr Ala Glu Glu Arg Tyr Pro
            100                 105                 110

Ile Leu Pro Glu Tyr Leu Gln Cys Val Lys Glu Leu Tyr Arg Gly Gly
        115                 120                 125

Leu Glu Pro Ile Asn Phe Gln Thr Ala Ala Asp Gln Ala Arg Glu Leu
    130                 135                 140

Ile Asn Ser Trp Val Glu Ser Gln Thr Asn Gly Ile Ile Arg Asn Val
145                 150                 155                 160

Leu Gln Pro Ser Ser Val Asp Ser Gln Thr Ala Met Val Leu Val Asn
                165                 170                 175

Ala Ile Val Phe Lys Gly Leu Trp Glu Lys Ala Phe Lys Asp Glu Asp
            180                 185                 190

Thr Gln Ala Met Pro Phe Arg Val Thr Glu Gln Glu Ser Lys Pro Val
        195                 200                 205

Gln Met Met Tyr Gln Ile Gly Leu Phe Arg Val Ala Ser Met Ala Ser
    210                 215                 220

Glu Lys Met Lys Ile Leu Glu Leu Pro Phe Ala Ser Gly Thr Met Ser
225                 230                 235                 240

Met Leu Val Leu Leu Pro Asp Glu Val Ser Gly Leu Glu Gln Leu Glu
                245                 250                 255

Ser Ile Ile Asn Phe Glu Lys Leu Thr Glu Trp Thr Ser Ser Asn Val
            260                 265                 270

Met Glu Glu Arg Lys Ile Lys Val Tyr Leu Pro Arg Met Lys Met Glu
        275                 280                 285

Glu Lys Tyr Asn Leu Thr Ser Val Leu Met Ala Met Gly Ile Thr Asp
    290                 295                 300

Val Phe Ser Ser Ser Ala Asn Leu Ser Gly Ile Ser Ser Ala Glu Ser
305                 310                 315                 320

Leu Lys Ile Ser Gln Ala Val His Ala Ala His Ala Glu Ile Asn Glu
                325                 330                 335

Ala Gly Arg Glu Val Val Gly Ser Ala Glu Ala Gly Val Asp Ala Ala
            340                 345                 350

Ser Val Ser Glu Glu Phe Arg Ala Asp His Pro Phe Leu Phe Cys Ile
        355                 360                 365

Lys His Ile Ala Thr Asn Ala
    370                 375
(2) INFORMATION FOR SEQUENCE IDENTIFICATION NUMBER: 6:
   (i) SEQUENCE CHARACTERISTICS:
      (A) LENGTH: 390
      (B) TYPE: amino acid
      (C) STRANDEDNESS:
      (D) TOPOLOGY: linear
   (xi) SEQUENCE DBSCRIPTION: SEQ ID NO: 6: 
Met Gln Met Ser Pro Ala Leu Thr Cys Leu Val Leu Gly Leu Ala Ile
1               5                   10                  15

Val Phe Gly Glu Gly Ser Ala Val His His Pro Pro Ser Tyr Val Ala
            20                  25                  30

His Leu Ala Ser Asp Phe Gly Val Arg Val Phe Gln Gln Val Ala Gln
        35                  40                  45

Ala Ser Lys Asp Arg Asn Val Val Phe Ser Pro Tyr Gly Val Ala Ser
    50                  55                  60

Val Leu Ala Met Leu Gln Leu Thr Thr Gly Gly Glu Thr Gln Gln Gln
65                  70                  75                  80

Ile Gln Ala Ala Met Gly Phe Lys Ile Asp Asp Lys Gly Met Ala Pro
                85                  90                  95

Ala Leu Arg His Leu Tyr Lys Glu Leu Met Gly Pro Trp Asn Lys Asp
            100                 105                 110

Glu Ile Ser Thr Thr Asp Ala Ile Phe Val Gln Arg Asp Leu Lys Leu
        115                 120                 125

Val Gln Gly Phe Met Pro His Phe Phe Arg Leu Phe Arg Ser Thr Val
    130                 135                 140

Lys Gln Val Asp Phe Ser Glu Val Glu Arg Ala Arg Phe Ile Ile Asn
145                 150                 155                 160

Asp Trp Val Lys Thr His Thr Lys Gly Met Ile Ser Asn Leu Leu Gly
                165                 170                 175

Lys Gly Ala Val Asp Gln Leu Thr Arg Leu Val Leu Val Asn Ala Leu
            180                 185                 190

Tyr Phe Asn Gly Gln Trp Lys Thr Pro Phe Pro Asp Ser Ser Thr His
        195                 200                 205

Arg Arg Leu Phe His Lys Ser Asp Gly Ser Thr Val Ser Val Pro Met
    210                 215                 220

Met Ala Gln Thr Asn Lys Phe Asn Tyr Thr Glu Phe Thr Thr Pro Asp
225                 230                 235                 240

Gly His Tyr Tyr Asp Ile Leu Glu Leu Pro Tyr His Gly Asp Thr Leu
                245                 250                 255

Ser Met Phe Ile Ala Ala Pro Tyr Glu Lys Glu Val Pro Leu Ser Ala
            260                 265                 270

Leu Thr Asn Ile Leu Ser Ala Gln Leu Ile Ser His Trp Lys Gly Asn
        275                 280                 285

Met Thr Arg Leu Pro Arg Leu Leu Val Leu Pro Lys Phe Ser Leu Glu
    290                 295                 300

Thr Glu Val Asp Leu Arg Lys Pro Leu Glu Asn Leu Gly Met Thr Asp
305                 310                 315                 320
 
Met Phe Arg Gln Phe Gln Ala Asp Phe Thr Ser Leu Ser Asp Gln Glu
                325                 330                 335
Pro Leu His Val Ala Gln Ala Leu Gln Lys Val Lys Ile Glu Val Asn
            340                 345                 350
Glu Ser Gly Thr Val Ala Ser Ser Ser Thr Ala Val Ile Val Ser Ala
        355                 360                 365
Arg Met Ala Pro Glu Glu Ile Ile Met Asp Arg Pro Phe Leu Phe Val
    370                 375                 380
Val Arg His Asn Pro Thr
385                 390
(2) INFORMATION FOR SEQUENCE IDENTIFICATION NUMBER: 7:
   (i) SEQUENCE CHARACTERISTICS:
      (A) LENGTH: 405
      (B) TYPE: amino acid
      (C) STRANDEDNESS:
      (D) TOPOLOGY: linear
   (xi) SEQUENCE DESCRIPTION: SEQ ID NO: 7: 
Met Glu Asp Leu Cys Val Ala Asn Thr Leu Phe Ala Leu Asn Leu Phe
1               5                   10                  15

Lys His Leu Ala Lys Ala Ser Pro Thr Gln Asn Leu Phe Leu Ser Pro
            20                  25                  30

Trp Ser Ile Ser Ser Thr Met Ala Met Val Tyr Met Gly Ser Arg Gly
        35                  40                  45

Ser Thr Glu Asp Gln Met Ala Lys Val Leu Gln Phe Asn Glu Val Gly
    50                  55                  60

Ala Asn Ala Val Thr Pro Met Thr Pro Glu Asn Phe Thr Ser Cys Gly
65                  70                  75                  80

Phe Met Gln Gln Ile Gln Lys Gly Ser Tyr Pro Asp Ala Ile Leu Gln
                85                  90                  95

Ala Gln Ala Ala Asp Lys Ile His Ser Ser Phe Arg Ser Leu Ser Ser
            100                 105                 110

Ala Ile Asn Ala Ser Thr Gly Asp Tyr Leu Leu Glu Ser Val Asn Lys
        115                 120                 125

Leu Phe Gly Glu Lys Ser Ala Ser Phe Arg Glu Glu Tyr Ile Arg Leu
    130                 135                 140

Cys Gln Lys Tyr Tyr Ser Ser Glu Pro Gln Ala Val Asp Phe Leu Glu
145                 150                 155                 160

Cys Ala Glu Glu Ala Arg Lys Lys Ile Asn Ser Trp Val Lys Thr Gln
                165                 170                 175

Thr Lys Gly Lys Ile Pro Asn Leu Leu Pro Glu Gly Ser Val Asp Gly
            180                 185                 190
 
Asp Thr Arg Met Val Leu Val Asn Ala Val Tyr Phe Lys Gly Lys Trp
        195                 200                 205

Lys Thr Pro Phe Glu Lys Lys Leu Asn Gly Leu Tyr Pro Phe Arg Val
    210                 215                 220

Asn Ser Ala Gln Arg Thr Pro Val Gln Met Met Tyr Leu Arg Glu Lys
225                 230                 235                 240

Leu Asn Ile Gly Tyr Ile Glu Asp Leu Lys Ala Gln Ile Leu Glu Leu
                245                 250                 255

Pro Tyr Ala Gly Asp Val Ser Met Phe Leu Leu Leu Pro Asp Glu Ile
            260                 265                 270

Ala Asp Val Ser Thr Gly Leu Glu Leu Leu Glu Ser Glu Ile Thr Tyr
        275                 280                 285

Asp Lys Leu Asn Lys Trp Thr Ser Lys Asp Lys Met Ala Glu Asp Glu
    290                 295                 300

Val Glu Val Tyr Ile Pro Gln Phe Lys Leu Glu Glu His Tyr Glu Leu
305                 310                 315                 320

Arg Ser Ile Leu Arg Ser Met Gly Met Glu Asp Ala Phe Asn Lys Gly
                325                 330                 335

Arg Ala Asn Phe Ser Gly Met Ser Glu Arg Asn Asp Leu Phe Leu Ser
            340                 345                 350

Glu Val Phe His Gln Ala Met Val Asp Val Asn Glu Glu Gly Thr Glu
        355                 360                 365

Ala Ala Ala Gly Thr Gly Gly Val Met Thr Gly Arg Thr Gly His Gly
    370                 375                 380

Gly Pro Gln Phe Val Ala Asp His Pro Phe Leu Phe Leu Ile Met His
385                 390                 395                 400

Lys Ile Thr Lys Cys
                405
(2) INFORMATION FOR SEQUENCE IDENTIFICATION NUMBER: 8:
   (i) SEQUENCE CHARACTERISTICS:
      (A) LENGTH: 375
      (B) TYPE: amino acid
      (C) STRANDEDNESS:
      (D) TOPOLOGY: linear
   (xi) SEQUENCE DESCRIPTION: SEQ ID NO: 8:

Met Glu Gln Leu Ser Thr Ala Asn Thr His Phe Ala Val Asp Leu Phe
1               5                   10                  15
Arg Ala Leu Asn Glu Ser Asp Pro Thr Gly Asn Ile Phe Ile Ser Pro
            20                  25                  30
Leu Ser Ile Ser Ser Ala Leu Ala Met Ile Phe Leu Gly Thr Arg Gly
        35                  40                  45

Asn Thr Ala Ala Gln Val Ser Lys Ala Leu Tyr Phe Asp Thr Val Glu
    50                  55                  60

Asp Ile His Ser Arg Phe Gln Ser Leu Asn Ala Asp Ile Asn Lys Pro
65                  70                  75                  80

Gly Ala Pro Tyr Ile Leu Lys Leu Ala Asn Arg Leu Tyr Gly Glu Lys
                85                  90                  95

Thr Tyr Asn Phe Leu Ala Asp Phe Leu Ala Ser Thr Gln Lys Met Tyr
            100                 105                 110

Gly Ala Glu Leu Ala Ser Val Asp Phe Gln Gln Ala Pro Glu Asp Ala
        115                 120                 125

Arg Lys Glu Ile Asn Glu Trp Val Lys Gly Gln Thr Glu Gly Lys Ile
    130                 135                 140

Pro Glu Leu Leu Val Lys Gly Met Val Asp Asn Met Thr Lys Leu Val
145                 150                 155                 160

Leu Val Asn Ala Ile Tyr Phe Lys Gly Asn Trp Gln Glu Lys Phe Met
                165                 170                 175

Lys Glu Ala Thr Arg Asp Ala Pro Phe Arg Leu Asn Lys Lys Asp Thr
            180                 185                 190

Lys Thr Val Lys Met Met Tyr Gln Lys Lys Lys Phe Pro Tyr Asn Tyr
        195                 200                 205

Ile Glu Asp Leu Lys Cys Arg Val Leu Glu Leu Pro Tyr Gln Gly Lys
    210                 215                 220

Glu Leu Ser Met Ile Ile Leu Leu Pro Asp Asp Ile Glu Asp Glu Ser
225                 230                 235                 240

Thr Gly Leu Glu Lys Ile Glu Lys Gln Leu Thr Leu Glu Lys Leu Arg
                245                 250                 255

Glu Trp Thr Lys Pro Glu Asn Leu Tyr Leu Ala Glu Val Asn Val His
            260                 265                 270

Leu Pro Arg Phe Lys Leu Glu Glu Ser Tyr Asp Leu Thr Ser His Leu
        275                 280                 285

Ala Arg Leu Gly Val Gln Asp Leu Phe Asn Arg Gly Lys Ala Asp Leu
    290                 295                 300

Ser Gly Met Ser Gly Ala Arg Asp Leu Phe Val Ser Lys Ile Ile His
305                 310                 315                 320

Lys Ser Phe Val Asp Leu Asn Glu Glu Gly Thr Glu Ala Ala Ala Ala
                325                 330                 335

Thr Ala Gly Thr Ile Met Leu Ala Met Leu Met Pro Glu Glu Asn Phe
            340                 345                 350

Asn Ala Asp His Pro Phe Ile Phe Phe Ile Arg His Asn Pro Ser Ala
        355                 360                 365

Asn Ile Leu Phe Leu Gly Arg
    370                 375